# EUROPEAN PATENT APPLICATION

(11) **EP 3 892 614 A1**
(43) Date of publication of application: **13.10.2021**
(21) Application number: 20315128.7
(22) Date of filing: 07.04.2020
(51) Int. Cl.: C07D 209/08, A61K 31/404, A61P 35/00

(54) **(1H-INDOL-5-YL)ACRYLAMIDE DERIVATIVES AS INHIBITORS OF TEAD PROTEINS AND THE HIPPO-YAP1/TAZ SIGNALING CASCADE FOR THE TREATMENT OF CANCER**

(71) Applicant: SANOFI, 75008 Paris (FR)
(72) Inventor: The designation of the inventor has not yet been filed
(74) Representative: Sanofi

(57) **Abstract**

The invention relates to 1H-indolyl-acrylamide derivatives of formula (I): 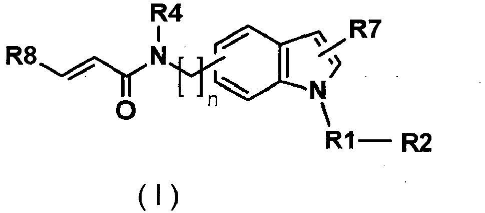 as inhibitors of the TEAD-dependent gene transcription and the HIPPO-YAP1/TAZ signaling cascade for use in methods of treatment of cancer, such as e.g. breast, ovarian, uterine, prostate, lung, gastric, colorectal, bladder, pancreatic and liver cancers, sarcomas, esophageal, head and neck cancers, uveal melanoma or glioma, and in particular in the treatment of patients who have exhibited resistance to prior anti-cancer therapy.
The present description discloses the syntheses of exemplary compounds as well as pharmacological data thereof (e.g. pages 11 to 48; examples 1 to 51; tables).
An exemplary compound is e.g. N-(1-(3-(trifluoromethyl)benzyl-1H-indol-5-yl)acrylamide ( example 1).

## Description

The present invention relates to indole compounds, to their preparation and to their therapeutic use.

The compounds described herein are inhibitors of YAP1/TAZ-TEAD or TEAD-dependent gene transcription.

### TEAD proteins and the HIPPO-YAP1/TAZ signaling cascade

Transcriptional enhanced associate domain (TEAD) proteins are transcription factors comprised of four family members (TEAD1-4) that function in modulating gene expression in response to the HIPPO pathway. TEAD proteins preferentially associate with transcription co-activators yes associated protein 1 (YAP1) or transcriptional co-activator with PDZ-binding motif (TAZ, also known as WWTR1). YAP1-TEAD or TAZ-TEAD bind to DNA and initiate the transcription of multiple different genes involved in cell proliferation, survival, mobility, stemness, and differentiation (reviewed in Holden and Cunningham, Cancer 2018). YAP1/TAZ-TEAD activity is tightly controlled by the HIPPO pathway.

The HIPPO pathway was initially discovered in Drosophila melanogaster as a key regulator of tissue growth. It is an evolutionarily conserved signaling pathway regulating numerous biological processes, including cell growth and fate decision, organ size control, and regeneration. The core of the Hippo pathway in mammals consists of a cascade of kinases including MST1/2 and LATS1/2, their associated adaptor proteins SAV1 and MOB1, as well as upstream regulators, such as NF2, SCRIBBLE, CRUMBS, and multiple G protein-coupled receptors. The Hippo pathway is tightly regulated by both intrinsic and extrinsic signals, such as mechanical force, cell-cell contact, polarity, energy status, stress, as well as many diffusible hormonal factors (reviewed in Ma et al., Annual Rev of Biochem 2019). Upon activation of Hippo pathway kinases (i.e. Hippo "on" state), cytosolic YAP1 and TAZ proteins are phosphorylated and therefore, remain inactive through sequestration in the cytoplasm and/or degradation by the proteasomal machinery. Upon inactivation of the Hippo pathway kinases (i.e. Hippo "off" state), cytosolic YAP1 and TAZ are not anymore phosphorylated and hence free to translocate into the cell nucleus, where they associate with TEAD transcription factors to bind DNA and regulate gene expression. Decreasing levels of pYAP1/YAP1 as well as increased expression of genes regulated by YAP1/TAZ-TEAD activity and increased promoter activity at TEAD-regulated genes are general indicators of YAP1 activation (reviewed in Totaro et Nature Cell Biol 2019).

### The Hippo-YAP1/TAZ/TEAD pathway and human cancer

In recent years, studies have demonstrated, that the deregulation of Hippo-YAP1TAZ-TEAD activity is at the origin of tumor progression and resistance to therapy in a number of different cancer indications and contexts. In mice, systematic genetic studies have clearly shown that either knocking out HIPPO pathway components (which are YAP1 inhibitors) or overexpressing YAP1 activators such as YAP1, TAZ, TEAD lead to YAP1 activation and YAP1-TEAD-dependent tumor initiation and tumor progression (Zhang et al, Dev Cell 2010; Lu et al, PNAS 2010; Nishio et al, PNAS 2015; Liu-Chittenden et al, Genes and Dev 2012). In humans, genetic alterations in the pathway are most prevalent for NF2 (neurofibromin), an upstream regulator of the core Hippo pathway, that has been linked to a heritable cancer syndrome and that has been classified as a tumor suppressor gene. Hundreds of somatically acquired mutations have been reported in NF2, predominantly in meningiomas, mesotheliomas and peripheral nerve sheath tumors, but also in other cancer types. (reviewed in Schroeder et Oncotarget 2013). Genetic alterations beyond NF2 and directly present within the core Hippo pathway are less frequently observed in patients and found at high prevalence only in certain indications such as, e.g. malignant mesothelioma. Malignant mesothelioma is a highly lethal cancer of serosal membranes and almost exclusively associated with asbestos exposure. It is a therapeutic indication that shows prevalent alterations in the HIPPO signaling pathway as well as high YAP1 activation and high dependence on YAP1-TEAD activity (reviewed in Sekido et al., Cancers 2018). Increased YAP1 or YAP1-TEAD activity is not limited to genetic alterations in the HIPPO pathway and can also be the result of upregulation through multiple interconnected signals. Numerous pathways with critical role in tumorigenesis feed into the HIPPO-YAP1/TAZ/TEAD1 cascade, well described examples include the RTK-RAS-RAF-MEK-ERK, WNT, TGF-beta, and AMPK pathways (reviewed in Han et J Transl Med 2019). The number of tumor types that depend at least in part on YAP1-TEAD activation is hence tremendous and spans from breast, ovarian, uterine, and prostate cancers to lung, gastric, colorectal, bladder, pancreatic, and liver cancers, and further to sarcomas, esophageal, head and neck cancers, uveal melanoma, and glioma (reviewed in Zanconato et Cancer Cell 2016). Recent studies reveal an interplay between the HIPPO-YAP/TAZ/TEAD pathway and the human immune response (reviewed in Yamauchi and Moroishi, Cells 2019).

YAP1 activation has been observed in the context of resistance to therapy and is recognized as a main mechanism of resistance and survival to anti-cancer treatment. In esophageal carcinoma, YAP1 is a positive regulator of EGFR (Epidermal Growth Factor Receptor) and the induction of YAP1 is associated with resistance to 5-FU and docetaxel. In the context of targeted therapies, YAP1 in BRAF-mutant tumors, acts as a parallel survival input to promote resistance to RAF and MEK inhibitor therapy in melanoma. Similarly, activation of YAP1 is a mechanism of survival to EGFR and MEK inhibitor treatment in the context of EGFR mutant lung cancer and multiple studies have identified YAP1 activation one of the main bypass mechanism to KRAS inhibition. In a hormone-dependent tumor context, TAZ inhibition was shown to restore sensitivity to tamoxifen in breast cancer. In prostate carcinoma cells, androgen deprivation therapy resistance was associated with increased YAP nuclear localization and activity (reviewed in Reggiani et 2020; Kurppa et Cancer Cell 2020).

Thus, the HIPPO-YAP/TAZ/TEAD pathway is a key player in cancer development and tumor maintenance and targeting this pathway will is key for cancer treatment, both in a first line therapy setting as well as in the context of overcoming drug resistance with multiple cancer indications.

Thereofre, there is a neeed for inhibitors of YAP1/TAZ-TEAD or TEAD-dependent gene transcription.

Disclosed herein are the compounds of the formula (I) 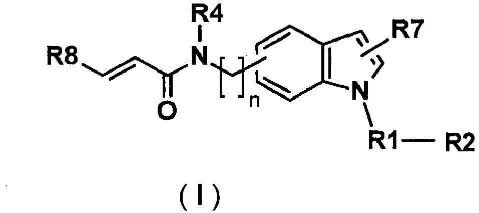 in which:
n is an integer chosen from 0 and 1
R1 is chosen from
   - a single bond, and
   - a (C1-C4) alkenyl group,
R2 is chosen from:
   - a (C1-C4) alkyl group or substituted with one or more fluorine atoms,
   - a (C1-C3) alkoxy group substituted with one or more fluorine atoms,
   - a phenyl group unsubstituted or substituted with one or more R3 groups,
   - a (C4-C8) cycloalkyl group unsubstituted or substituted with one or more R5 groups,
   - a (C4-C8) heterocyclyl group unsubstituted or substituted with one or more R6 groups, and
   - a NR9R10 group,
R3 is chosen from a
   - a (C1-C4) alkyl group unsubstituted or substituted with one or more fluorine atoms,
   - a cyclopropyl group,
   - a halogen atom,
   - a (C1-C3) alkoxy group unsubstituted or substituted with one or more fluorine atoms,
   - a pentafluorosulfanyl group,
   - a nitrile group,
   - a (C1-C3) trialkylsilyl group,
   - a (C1-C3) aklylsulfonyl group, and
   - a phenyl group unsubstituted or substituted with a trifluoromethyl group,
R4 is chosen from a hydrogen atom and a (C1-C4) alkyl group,
R5 is chosen from a fluorine atom and a trifluoromethyl group,
R6 is chosen from
   - a phenyl group unsubstituted or substituted with one or more fluorine atoms or one or more CF3 groups,
   - a (C1-C4) alkyl group substituted with one or more fluorine atoms, and
   - a fluorine atom,
R7 is chosen from
   - a hydrogen atom,
   - a nitrile group, and
   - a (C1-C4) alkyl group,
R8 is chosen from a hydrogen atom and a (C1-C4) alkyl group unsubstituted or substituted with a di(C1-C4) alkylamino group,
R9 and R10 are identical or different and chosen from an (C1-C3) alkyl group unsubstituted or substituted with one or more fluorine atoms,
or a pharmaceutically acceptable salt thereof.

The compounds of formula (I) may comprise one or more asymmetric carbon atoms. They may thus exist in the form of enantiomers or diastereoisomers and also mixtures thereof.

The compounds of formula (I) may be present as well under tautomer forms.

The compounds of formula (I) may exist in the form of bases or addition salts with acids or bases, in particular pharmaceutically acceptable salts.

Pharmaceutically acceptable salts of the compounds of formula (I) do form part of the invention.

As used herein, certain terms have the following definitions:
- a halogen atom: a fluorine, a chlorine, a bromine or an iodine atom;
- an alkyl group: a linear or branched saturated aliphatic group. Examples include the groups methyl, ethyl, propyl, isopropyl, etc;
- a cycloalkyl group: a cyclic alkyl group, including spiro groups. Examples include the groups cyclopropyl, cyclobutyl, cyclopentyl, cyclohexyl, spiro[2.3]hexanyl, etc.;
- an alkenyl group: a linear or branched mono- or polyunsaturated aliphatic group containing, for example, one or two ethylenic unsaturations. Examples include the -CH2- or methylene group;
- an alkoxy group: a radical -O-alkyl in which the alkyl group is as defined above.

Examples of alkoxy group include methoxy, ethoxy, isopropoxy, etc.;
- a nitrile group: a group CN;
- a hydroxy group: a group OH;
- an aryl group: a cyclic aromatic group comprising between 5 and 10 carbon atoms.

Examples of an aryl group include phenyl group;
- a heterocyclyl group: a saturated or insaturated or cyclic group containing between 4 and 9 carbon atoms, and containing 1 or 2 heteroatoms, such as oxygen or nitrogen. Examples of heterocyclyl groups include pyrrolidine, piperazine, piperidine, tetrahydropyran, morpholine and diazepane groups;
- an amino group: a group -NH2;
- a dialkylamino group: an amino group substituted with two alkyl groups;
- a sulfonyl group: a substituted SO2 group; and
- a silyl group: a group containing a silicium atom. Example of silyl groups includes trimethylsilyl group.

In one aspect, the compounds of formula (I) comprise a first group of compounds of the following formula: 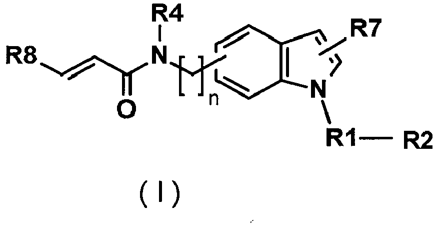 or a pharmaceutically acceptable salt thereof.

In another aspect, the compounds of formula (I) comprise a second group composed of the compounds in which R4 is a hydrogen atom.

In another aspect, the compounds of formula (I) comprise a third group composed of the compounds in which R7 is a hydrogen atom.

In another aspect, the compounds of formula (I) comprise a fourth group composed of the compounds in which R8 is a hydrogen atom.

In another aspect, the compounds of formula (I) comprise a fifth group composed of the compounds in which:
n is 0,
R1 is a single bond;
R2 is chosen from:
   - a phenyl group unsubstituted or substituted with one or more R3 groups,
   - a (C4-C8) cycloalkyl group unsubstituted or substituted with one or more R5 groups, and
   - a (C4-C8) heterocyclyl group unsubstituted or substituted with one or more R6 groups.

Among the compounds of the fifth group, mention may be made of the compounds of formula (I) for which R2 is a phenyl group substituted with one or more R3 groups.

Among the compounds of the latter groups, mention may be made of the compounds of formula (I) for which R3 is a (C1-C4) alkyl group substituted with one or more fluorine atoms, in particular a trifluoromethyl group.

In another aspect, the compounds of formula (I) comprise a sixth group composed of the compounds for which R1 is chosen from a single bond and a methylene group.

In another aspect, the compounds of formula (I) comprise a seventh group composed of the compounds for which R2 is chosen from:
- a (C1-C4) alkyl group or substituted with three fluorine atoms,
- a (C1-C3) alkoxy group substituted with three fluorine atoms, in particular a trifluoroethoxy group,
- a phenyl group unsubstituted or substituted with one or more R3 groups,
- a (C4-C8) cycloalkyl group, in particular a cyclobutyl, a cyclopentyl, a cyclohexyl or a spiro[2.3]hexanyl, unsubstituted or substituted with one or more R5 groups,
- a (C4-C8) heterocyclyl group, in particular a tetrahydrofuranyl, a tetrahydropyranyl, a piperidinyl, a pyrrolidinyl or a pyridinyl group, unsubstituted or substituted with one or more R6 groups, and
- a NR9R10 group,

In another aspect, the compounds of formula (I) comprise an eighth group composed of the compounds for which R3 is chosen from:
- a methyl group or a (C1-C4) trifluoroalkyl group, in particular a trifluoromethyl group,
- a cyclopropyl group,
- an iodine or a fluorine atom,
- a methoxy group or a trifluoromethoxy group,
- a pentafluorosulfanyl group,
- a nitrile group,
- a trimethylsilyl group,
- a methylsulfonyl group, and
- a phenyl group unsubstituted or substituted with a trifluoromethyl group,

In another aspect, the compounds of formula (I) comprise a ninth group composed of the compounds for which R4 is chosen from a hydrogen atom and a methyl group.

Among the compounds of the latter groups, mention may be made of the compounds of formula (I) for which R4 is a hydrogen atom.

In another aspect, the compounds of formula (I) comprise a tenth group composed of the compounds for which R6 is chosen from:
- a phenyl group unsubstituted or substituted with three fluorine atoms or a trifluoromethyl group,
- a (C1-C4) alkyl group substituted with three fluorine atoms, in particular a trifluoromethyl or a trifluoroethyl group, and
- a fluorine atom.

In another aspect, the compounds of formula (I) comprise an eleventh group composed of the compounds for which R7 is chosen from a hydrogen atom, a nitrile atom or a methyl group.

In another aspect, the compounds of formula (I) comprise a twelfth group composed of the compounds for which R8 is a hydrogen atom or a methyl group substituted with a dimethylamino group.

In another aspect, the compounds of formula (I) comprise a thirteenth group composed of the compounds for which R9 and R10 or chosen from a methyl group and a trifluoroethyl group.

All these sub-groups taken alone or in combination are part of the description.

Among the compounds of formula (I) that are subjects matter of the invention, mention may be made in particular of the following compounds:
N-[1-[[3-(trifluoromethyl)phenyl]methyl]indol-5-yl]prop-2-enamide
N-methyl-N-(1-(3-(trifluoromethyl)benzyl)-1 H-indol-5-yl)acrylamide
N-(1-(5,5,5-trifluoropentyl)-1H-indol-5-yl)acrylamide
N-(1-(1-(3-(trifluoromethyl)phenyl)ethyl)-1H-indol-5-yl)acrylamide
N-(1-(3-(pentafluoro-lambda6-sulfanyl)benzyl)-1H-indol-5-yl)acrylamide
N-(1-(4-(pentafluoro-lambda6-sulfanyl)benzyl)-1H-indol-5-yl)acrylamide
N-(1-(4-(trifluoromethoxy)benzyl)-1H-indol-5-yl)acrylamide
N-(1-(3-(trifluoromethoxy)benzyl)-1H-indol-5-yl)acrylamide
N-(1-(3-iodobenzyl)-1H-indol-5-yl)acrylamide
N-(1-(2-fluoro-5-(trifluoromethyl)benzyl)-1H-indol-5-yl)acrylamide
N-(3-methyl-1-(3-(trifluoromethyl)benzyl)-1 H-indol-5-yl)acrylamide
N-(1-(3-methoxybenzyl)-1H-indol-5-yl)acrylamide
N-(2-methyl-1-(3-(trifluoromethyl)benzyl)-1 H-indol-5-yl)acrylamide
N-(3-cyano-1-(3-(trifluoromethyl)benzyl)-1H-indol-5-yl)acrylamide
N-(1-(3-cyclopropylbenzyl)-1H-indol-5-yl)acrylamide
N-(2,3-dimethyl-1-(3-(trifluoromethyl)benzyl)-1 H-indol-5-yl)acrylamide
N-(1-(3-methylbenzyl)-1H-indol-5-yl)acrylamide
N-(1-Benzyl-1H-indol-5-yl)-acrylamide
N-(1-(3-(trifluoromethyl)benzyl)-1H-indol-4-yl)acrylamide (E)-4-(dimethylamino)-N-(1-(3-(trifluoromethyl)benzyl)-1H-indol-4-yl)but-2-enamide
N-(1-(3-(trifluoromethyl)benzyl)-1H-indol-6-yl)acrylamide
N-(1-((4,4-difluorocyclohexyl)methyl)-1H-indol-5-yl)acrylamide
N-(1-((tetrahydro-2H-pyran-4-yl)methyl)-1H-indol-5-yl)acrylamide
N-(1-((4-(trifluoromethyl)cyclohexyl)methyl)-1H-indol-5-yl)acrylamide
N-(1-((1,1-difluorospiro[2.3]hexan-5-yl)methyl)-1H-indol-5-yl)acrylamide
N-(1-((3-fluorocyclopentyl)methyl)-1H-indol-5-yl)acrylamide
N-(1-(cyclohexylmethyl)-1H-indol-5-yl)acrylamide
N-(1-((3-(trifluoromethyl)cyclobutyl)methyl)-1H-indol-5-yl)acrylamide
N-(1-((3,3-difluorocyclopentyl)methyl)-1H-indol-5-yl)acrilamide
N-(1-([1,1'-biphenyl]-4-ylmethyl)-1H-indol-5-yl)acrylamide
N-(1-((3'-(trifluoromethyl)-[1,1'-biphenyl]-4-yl)methyl)-1H-indol-5-yl)acrylamide
N-(1-((4'-(trifluoromethyl)-[1,1'-biphenyl]-4-yl)methyl)-1H-indol-5-yl)acrylamide
N-(1-((trans)-4-(trifluoromethyl)cyclohexyl)-1H-indol-5-yl)acrylamide
N-(1-((cis)-4-(trifluoromethyl)cyclohexyl)-1H-indol-5-yl)acrylamide
N-(1-((1-(4-(trifluoromethyl)phenyl)piperidin-4-yl)methyl)-1H-indol-5-yl)acrylamide
N-(1-(2-(methyl(2,2,2-trifluoroethyl)amino)ethyl)-1H-indol-5-yl)acrylamide
N-(1-(2-(2,2,2-trifluoroethoxy)ethyl)-1H-indol-5-yl)acrylamide
N-(1-(1-(2,2,2-trifluoroethyl)piperidin-4-yl)-1H-indol-5-yl)acrylamide
N-(1-(4,4-difluorocyclohexyl)-1H-indol-5-yl)acrylamide
N-(1-(1-(4-(trifluoromethyl)phenyl)piperidin-4-yl)-1H-indol-5-yl)acrylamide
N-(1-(3-(3,3-difluoropyrrolidin-1-yl)propyl)-1H-indol-5-yl)acrylamide
N-(1-(4-(trimethylsilyl)benzyl)-1H-indol-5-yl)acrylamide
N-(1-(3-(trifluoromethyl)phenyl)-1H-indol-5-yl)acrylamide
N-(1-(4-(trifluoromethyl)phenyl)-1H-indol-5-yl)acrylamide
N-(1-(6-(trifluoromethyl)pyridin-3-yl)-1H-indol-5-yl)acrylamide
N-(1-(5-(trifluoromethyl)pyridin-2-yl)-1H-indol-5-yl)acrylamide
N-(1-(pyridin-3-yl)-1H-indol-5-yl)acrylamide
N-(1-(4-fluorophenyl)-1H-indol-5-yl)acrylamide
N-(1-(3,4-difluorophenyl)-1H-indol-5-yl)acrylamide
N-(1-(3-(methylsulfonyl)phenyl)-1H-indol-5-yl)acrylamide
N-(1-(4-cyanophenyl)-1H-indol-5-yl)acrylamide
or a pharmaceutically acceptable salt thereof.

The compounds of formula (I) may be prepared according to the processes that follow. Unless otherwise mentioned, n and R1 to R10 are as defined previously.

In the following Schemes, the starting compounds and the reagents, when their preparation method is not described, are commercially available or described in the literature, or else may be prepared according to methods that are described therein or that are known to those skilled in the art.

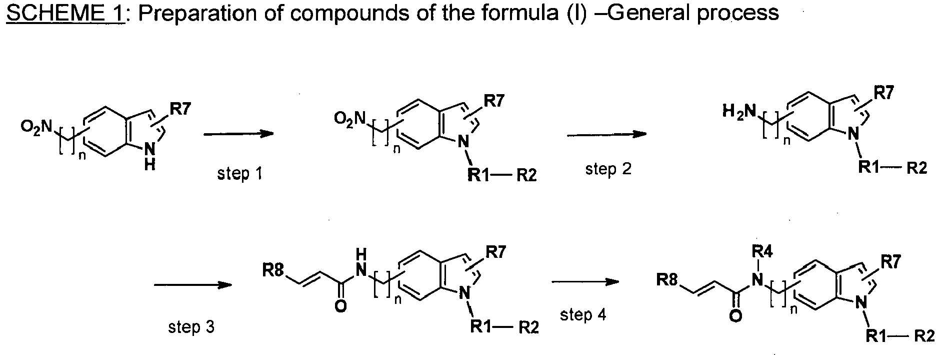

All compounds described hereinafter can be synthesized according to scheme 1.
Step 1 consists in an alkylation procedure of the indole nitrogen (by use the of corresponding alcools via Mitsunobu-reaction or the corresponding halogen-alkane derivatives, which themselves can be functionalized in further steps) except for R1 = bond, where the corresponding iodo-aryl derivatives were used for a direct arylation.
Step 2 represents a reduction of the nitro-group followed in step 3 by an amide formation using the appropriate acryloyl chloride or acrylic acid derivatives.

The alkylation of the amide in step 4 is realized by the use of aliphatic halogenoalkane derivatives.

The examples that follow describe the preparation of certain compounds of formula (I). The examples are not limiting but serve merely to illustrate the present invention.

The following abbreviations and empirical formulae are used:
- AcOEt: ethylacetate
- CMBP: Cyanomethylene-tributylphosphorane
- DCM: dichloromethane
- DMF: N,N-dimethylformamide
- DMSO: dimethylsulfoxide
- DPPF: 1,1'-bis(diphenylphosphino)ferrocene]dichloropalladium(II)
- PdCl2(PPh3)2: 1,1'-bis(triphenylphosphine)-palladium(II)chloride
- Et3N: triethylamine
- FA: formic acid
- MeCN: acetonitrile
- MeOH: methanol
- RT: room temperature
- Tr: Retention time
- °C: degree Celsius
- ml: milliliter(s)
- mmol: millimole(s)
- min: minute(s)
- µmol: micromole(s)
- µl: microliter(s)
- h: hour(s)

Some representative examples of the synthesis of the compounds of formula (I) are described in the following schemes.

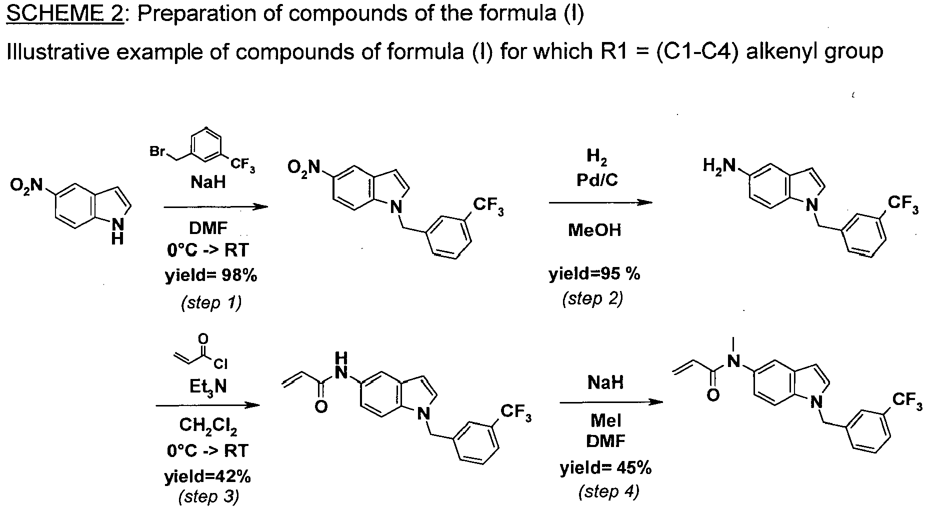

### 5-nitro-1-(3-(trifluoromethyl)benzyl)-1H-indole (step 1 of scheme 1)

To a solution of **5-nitroindole** (500 mg, 3.02 mmol) in 5ml DMF at 0 °C was added sodium hydride 60% in mineral oil (121 mg, 3.02 mmol) in several fractions, the mixture was allowed to rise at RT, stirred for 20 min at RT and cooled again to 0 °C before pouring drop by drop 3-(trifluoromethyl) benzyl bromide (737 mg, 3.02 mmol) solubilized in 2 ml of DMF. The solution was allowed to rise to RT and stirred for further 3h at RT. The crude mixture was poured on ice and water, extracted with AcOEt, dried over Na₂SO₄, evaporated at reduced pressure to obtain 0.95 g of **5-nitro-1-(3-(trifluoromethyl)-benzyl)-1H-indole** as a yellow solid, yield = 98%.

### 1-(3-(trifluoromethyl)benzyl)-1H-indol-5-amine (step 2 of scheme 1)

**5-nitro-1-(3-(trifluoromethyl)benzyl)-1H-indole** (1 g, 3.12 mmol) obtained in the preceding step was solubilized in 30 ml of MeOH and 3ml of AcOEt, Pd/C 10% (50% wet) (30 mg 160.0 µmol) was added and the mixture was hydrogenated in a Parr apparatus at RT under 30psi hydrogen pressure for 3h. After filtration and evaporation at reduced pressure 858 mg of a slightly brownish coloured oil of were obtained and used without further purification in the next step, yield 95%.

### Example 1 N-(1-(3-(trifluoromethyl)benzyl)-1H-indol-5-yl)acrylamide

**1-(3-(trifluoromethyl)benzyl)-1H-indol-5-amine** (150 mg, 517 µmol) obtained in the step described above were solubilized in dichloromethane (2 ml) with Et₃N (86 µl, 620 µmol), cooled down to 0°C before adding acryloyl chloride (53 µl, 620 µmol). Stirring was continued for 2h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain 75 mg of **N-(1-(3-(trifluoromethyl)benzyl)-1H-indol-5-yl)acrylamide** as a white solid, yield 42%

### Example 2 N-methyl-N-(1-(3-(trifluoromethyl)benzyl-1H-indol-5-yl)acrylamide

**N-(1-(3-(trifluoromethyl)benzyl)-1H-indol-5-yl)acrylamide** (270 mg, 784 µmol) obtained in example 1 was solubilized in 2ml DMF, cooled to 0°C before adding sodium hydride 60% in mineral oil (31 mg, 784 µmol), the suspension was stirred 20 min at 0°C before iodomethane (49 µl, 784 µmol) was added and the mixture was stirred 3h at RT, poured into water, extracted with AcOEt, washed with water and an aqueous saturated solution of NaCl, dried over Na₂SO₄. After purification by reverse phase chromatography (C18, Water/CH₃CN, gradient 95/5 to 5/95) 130 mg **of N-methyl-N-(1-(3-(trifluoromethyl)-benzyl)-1H-indol-5-yl)acrylamide** were isolated as a colorless oil, yield 45%.

The following examples 3 - 29 (analogues of example 1) were synthesized using the same reaction sequence as in scheme 2, using the appropriate indoles, bromoalkyl and acrylic acid compounds as starting materials, which are known to those skilled in the art. Described hereinbelow are steps 3 of scheme 1.

### Example 3 N-(1-(5,5,5-trifluoropentyl)-1H-indol-5-yl)acrylamide

**1-(5,5,5-trifluoropentyl)-1H-indol-5-amine** (200 mg, 780 µmol) were solubilized in dichloromethane (5 ml) with Et₃N (131 µl, 937 µmol), cooled down to 0°C before adding acryloyl chloride (88 mg, 937 µmol). Stirring was continued for 1h30 at RT. The reaction mixture was diluted with dichloromethane, extracted with water, dried over Na₂SO₄, evaporated at reduced pressure and purified by column chromatography (silica gel, gradient heptane/AcOEt 100/0 to 70/30) then by reverse phase chromatography (C18, water/MeCN, gradient 95/5 to 5/95) to obtain 23 mg of N-(1-(5,5,5-trifluoropentyl)-1H-indol-5-yl)acrylamide as a brown oil, yield 10%.

### Example 4 N-(1-(1-(3-(trifluoromethyl)phenyl)ethyl)-1H-indol-5-yl)acrylamide

**1-(1-(3-(trifluoromethyl)phenyl)ethyl)-1H-indol-5-amine** (200 mg, 657 µmol) were solubilized in dichloromethane (5 ml) with Et₃N (110 µl, 789 µmol), cooled down to 0°C before adding acryloyl chloride (74 mg, 789 µmol). Stirring was continued for 3h at RT. The reaction mixture was diluted with dichloromethane, extracted with water, dried over Na₂SO₄, evaporated at reduced pressure and purified by column chromatography (silica gel, gradient heptane/AcOEt, 100/0 to 75/25) to obtain 66 mg of N-(1-(1-(3-(trifluoromethyl)phenyl)ethyl)-1H-indol-5-yl)acrylamide as a white solid after trituration in diethylether, yield 28%.

### Example 5 N-(1-(3-(pentafluoro-lambda6-sulfanyl)benzyl)-1H-indol-5-yl)acrylamide

**1-(3-(pentafluoro-lambda6-sulfanyl)benzyl)-1H-indol-5-amine** (200 mg, 574 µmol) were solubilized in dichloromethane (5 ml) with Et₃N (96 µl, 689 µmol), cooled down to 0°C before adding acryloyl chloride (65 mg, 689 µmol). Stirring was continued for 3h at RT. The reaction mixture was diluted with dichloromethane, extracted with water, dried over Na₂SO₄, evaporated at reduced pressure and purified by column chromatography (silica gel, gradient heptane/AcOEt, 100/0 to 70/30) to obtain 80 mg of **N-(1-(3-(pentafluoro-lambda6-sulfanyl)benzyl)-1H-indol-5-yl)acrylamide** as a white solid after trituration in diethylether, yield 34%.

### Example 6 N-(1-(4-(pentafluoro-lambda6-sulfanyl)benzyl)-1H-indol-5-yl)acrylamide

**1-(4-(pentafluoro-lambda6-sulfanyl)benzyl)-1H-indol-5-amine** (200 mg, 574 µmol) were solubilized in dichloromethane (5 ml) with Et₃N (120 µl, 861 µmol), cooled down to 0°C before adding acryloyl chloride (65 mg, 689 µmol). Stirring was continued for 1^st RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain 98 mg of **N-(1-(4-(pentafluoro-lambda6-sulfanyl)benzyl)-1H-indol-5-yl)acrylamide**as a white solid after trituration in diethylether, yield 42%.

### Example 7 N-(1-(4-(trifluoromethoxy)benzyl)-1H-indol-5-yl)acrylamide

**1-(4-(trifluoromethoxy)benzyl)-1H-indol-5-amine** (200 mg, 653 µmol) were solubilized in dichloromethane (7 ml) with Et₃N (192 µl, 1.31mmol), cooled down to 0°C before adding acryloyl chloride (66 µl, 784 µmol). Stirring was continued for 1h at RT. The reaction mixture was diluted with dichloromethane, extracted with water, dried over Na₂SO₄, evaporated at reduced pressure and purified by column chromatography (silica gel, gradient heptane/AcOEt, 100/0 to 70/30) to obtain 87 mg of **N-(1-(4-(trifluoro-methoxy)-benzyl)-1H-indol-5-yl)acrylamide** as an amorphous solid, yield 42.5%.

### Example 8 N-(1-(3-(trifluoromethoxy)benzyl)-1H-indol-5-yl)acrylamide

**1-(3-(trifluoromethoxy)benzyl)-1H-indol-5-amine** (200 mg, 653 µmol) were solubilized in dichloromethane (7ml) with Et₃N (192 µl, 1.31 mmol), cooled down to 0°C before adding acryloyl chloride (66 µl, 784 µmol). Stirring was continued for 1h at RT. The reaction mixture was diluted with dichloromethane, extracted with water, dried over Na₂SO₄, evaporated at reduced pressure and purified by column chromatography (silica gel, gradient heptane/AcOEt, 100/0 to 70/30) to obtain 100 mg of **N-(1-(3-(trifluoro-methoxy)-benzyl)-1H-indol-5-yl)acrylamide** as an amorphous solid, yield 42%.

### Example 9 N-(1-(3-iodobenzyl)-1H-indol-5-yl)acrylamide

**1-(3-iodobenzyl)-1H-indol-5-amine** (280 mg, 745 µmol) were solubilized in dichloro-methane (7ml) with Et₃N (220 µl, 1,50 mmol), cooled down to 0°C before adding acryloyl chloride (73 µl, 900 µmol). Stirring was continued for 1h at RT. The reaction mixture was diluted with dichloromethane, extracted with water, dried over Na₂SO₄, evaporated at reduced pressure and purified by column chromatography (silica gel, gradient heptane/AcOEt 100/0 to 70/30) to obtain 90 mg of **N-(1-(3-iodobenzyl)-1H-indol-5-yl)acrylamide** as an amorphous solid, yield 24%.

### Example 10 N-(1-(2-fluoro-5-(trifluoromethyl)benzyl)-1H-indol-5-yl)acrylamide

**1-(2-fluoro-5-(trifluoromethyl)benzyl)-1H-indol-5-amine** (200 mg, 645 µmol) were solubilized in dichloromethane (5 ml) with Et₃N (118 µl, 844 µmol), cooled down to 0°C before adding acryloyl chloride (74 mg, 779 µmol). Stirring was continued for 3h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain 49 mg of **N-(1-(2-fluoro-5-(trifluoromethyl)benzyl)-1H-indol-5-yl)acrylamide** as a beige solid, yield 21%.

### Example 11 N-(3-methyl-1-(3-(trifluoromethyl)benzyl)-1H-indol-5-yl)acrylamide

**3-methyl-1-(3-(trifluoromethyl)benzyl)-1H-indol-5-amine** (200 mg, 657 µmol) were solubilized in dichloromethane (5 ml) with Et₃N (110 µl, 789 µmol), cooled dow to 0°C before adding acryloyl chloride (74 mg, 789 µmol). Stirring was continued for 2h at RT. The reaction mixture was diluted with CH₂Cl₂, extracted with water, dried over Na₂SO₄, evaporated at reduced pressure and purified by column chromatography (silica gel, gradient heptane/AcOEt 100/0 to 50/50) to obtain 28 mg of **N-(1N-(3-methyl-1-(3-(trifluoromethyl)benzyl)-1H-indol-5-yl)acrylamide** as a white solid after trituration in diethylether, yield 11%.

### Example 12 N-(1-(3-methoxybenzyl)-1H-indol-5-yl)acrylamide

**1-(3-methoxybenzyl)-1H-indol-5-amine** (227 mg, 900 µmol) were solubilized in dichloromethane (5 ml) with Et₃N (190µl, 1.08 mmol), cooled down to 0°C before adding acryloyl chloride (80 µl, 950 µmol). Stirring was continued for 40 min at 0°C. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain after trituration in AcOEt 45 mg of **N-(1-(3-methoxybenzyl)-1H-indol-5-yl)acrylamide** as a white solid, yield 16%.

### Example 13 N-(2-methyl-1-(3-(trifluoromethyl)benzyl)-1H-indo)-5-yl)acrylamide

**2-methyl-1-(3-(trifluoromethyl)benzyl)-1H-indol-5-amine** (200 mg, 657 µmol) were solubilized in dichloromethane (2 ml) with Et₃N (138 µl, 986 µmol), cooled down to 0°C before adding acryloyl chloride (74 mg, 789 µmol). Stirring was continued for 3h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain after trituration in diethylether 30 mg of **N-(2-methyl-1-(3-(trifluoromethyl)benzyl)-1H-indol-5-yl)acrylamide** as a white solid, yield 12%.

### Example 14 N-(3-cyano-1-(3-(trifluoromethyl)benzyl)-1H-indo)-5-yl)acrylamide

**5-amino-1-(3-(trifluoromethyl)benzyl)-1H-indole-3-carbonitrile** (230 mg, 730 µmol) were solubilized in dichloromethane (2 ml) with Et₃N (153 µl, 1,09 mmol)), cooled down to 0°C before adding acryloyl chloride (83 mg, 875 µmol). Stirring was continued for 1h30 at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain after trituration in dichloromethane 69 mg of **N-(3-cyano-1-(3-(trifluoromethyl)benzyl)-1H-indol-5-yl)-acrylamide** as a beige solid, yield 25%.

### Example 15 N-(1-(3-cyclopropylbenzyl)-1H-indol-5-yl)acrylamide

1-(3-cyclopropylbenzyl)-1H-indol-5-amine (170 mg, 647 µmol) were solubilized in dichloromethane (2.5 ml) with Et₃N (108 µl, 778 µmol) cooled down to 0°C before adding acryloyl chloride (60 µl, 713 µmol). Stirring was continued for 30 min at 0°C. The reaction mixture was diluted with dichloromethane, extracted with water, dried over Na₂SO₄, evaporated at reduced pressure and purified by column chromatography (silica gel, gradient heptane/AcOEt 95/5 to 60/40) to obtain after trituration in diethylether 40 mg of N-**(1-(3-cyclopropylbenzyl)-1H-indol-5-yl)acrylamide** as a white solid, yield 20%.

### Example 16 N-(2,3-dimethyl-1-(3-(trifluoromethyl)benzyl)-1H-indol-5-yl)acrylamide

**2,3-dimethyl-1-(3-(trifluoromethyl)benzyl)-1H-indol-5-amine** (200 mg, 628 µmol) were solubilized in dichloromethane (2 ml) with Et₃N (131 µl, 942 µmol), cooled down to 0°C before adding acryloyl chloride (71 mg, 754 µmol). Stirring was continued for 2h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain after trituration in diethylether 33 mg of **N-(2,3-dimethyl-1-(3-(trifluoromethyl)benzyl)-1H-indol-5-yl)acrylamide** as a beige solid, yield 14%.

### Example 17 N-(1-(3-methylbenzyl)-1H-indol-5-acrylamide

**1-(3-methylbenzyl)-1H-indol-5-amine** (388 mg, 1.64 mmol) were solubilized in dichloromethane (16 ml) with Et₃N (344 µl, 1.97 mmol) cooled down to 0°C before adding acryloyl chloride (160 µl, 1.97 mmol). Stirring was continued for 45 min at 0°C. The reaction mixture was diluted with dichloromethane, extracted with water, dried over Na₂SO₄, evaporated at reduced pressure and purified by column chromatography (silica gel, gradient cyclohexane/AcOEt 100/0 to 50/50) to obtain 252 mg of **N-(1-(3-methyl-benzyl)-1H-indol-5-yl)-acrylamide** as a pink solid, yield 53%.

### Example 18 N-(1-Benzyl-1H-indol-5-yl)-acrylamide

**1-benzyl-1H-indol-5-amine** (30 mg, 140 µmol) were solubilized in dichloromethane (2 ml) with Et₃N (28 µl, 160 µmol) cooled down to 0°C before adding acryloyl chloride (13 µl, 160 µmol). Stirring was continued for 45 min at 0°C. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain 6 mg of **N-(1-Benzyl-1H-indol-5-yl)-acrylamide** as a solid, yield 16%.

### Example 19 N-(1-(3-(trifluoromethyl)benzyl)-1H-indol-4-yl)acrylamide

**1-(3-(trifluoromethyl)benzyl)-1H-indol-4-amine** (150 mg, 517 µmol) were solubilized in dichloromethane (2 ml) with Et₃N (108 µl, 776 µmol), cooled down to 0°C before adding acryloyl chloride (56 mg, 621 µmol). Stirring was continued for 3h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain after trituration in diethylether 95 mg of **N-(1-(3-(trifluoromethyl)benzyl)-1H-indol-4-yl)acrylamide** as a white solid, yield 51%.

### Example 20 (E)-4-(dimethylamino)-N-(1-(3-(trifluoromethyl)benzyl)-1H-indol-4-yl)but-2-enamide

**(E)-4-dimethylaminocrotonic acid hydrochloride** (176 mg, 1.03 mmol) were suspended in dichloromethane (8 ml) with a catalytic amount of DMF (11 µl, 138 µmol), cooled down to 0°C before adding oxalyl chloride (121 µl, 1.38 mmol). Stirring was continued for 2h at RT. The reaction mixture was evaporated at reduced pressure. The crude (E)-4-(dimethylamino)but-2-enoyl chloride was used as that in the further step:
**1-(3-(trifluoromethyl)benzyl)-1H-indol-4-amine** (200 mg, 689 µmol) was solubilized in dichloromethane (5 ml) with pyridine (223 µl, 2.76 mmol), cooled down to 0°C before adding the previously prepared (E)-4-(dimethylamino)but-2-enoyl chloride diluted in dichloromethane (5ml). Stirring was continued for 3h at RT. The reaction mixture was diluted with dichloromethane, extracted with water, dried over Na₂SO₄, evaporated at reduced pressure and purified by column chromatography (silica gel, gradient CH₂Cl₂/MeOH 100/0 to 95/5) to obtain 76 mg of **(E)-4-(dimethylamino)-N-(1-(3-(trifluoromethyl)benzyl)-1H-indol-4-yl)but-2-enamide** as a white solid, yield 27%.

### Example 21 N-(1-(3-(trifluoromethyl)benzyl)-1H-indol-6-yl)acrylamide

**1-(3-(trifluoromethyl)benzyl)-1H-indol-6-amine** (150 mg, 517 µmol) were solubilized in dichloromethane (2 ml) with Et₃N (94 µl, 672 µmol), cooled down to 0°C before adding acryloyl chloride (59 mg, 620 µmol). Stirring was continued for 4h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain 110 mg of **N-(1-(3-(trifluoromethyl)-benzyl)-1H-indol-6-yl)acrylamide** as a white solid, yield 62%.

### Example 22 N-(1-((4,4-difluorocyclohexyl)methyl)-1H-indol-5-yl)acrylamide

**1-((4,4-difluorocyclohexyl)methyl)-1H-indol-5-amine** (101 mg, 382 µmol) were solubilized in dichloromethane (4 ml) with Et₃N (80 µl, 459 µmol) cooled down to 0°C before adding acryloyl chloride (38 µl, 459 µmol). Stirring was continued for 45 min at 0°C. The reaction mixture was diluted with dichloromethane, extracted with water, dried over Na₂SO₄, evaporated at reduced pressure and purified by column chromatography (silica gel, gradient cyclohexane/AcOEt 100/100 to 50/50) to obtain 61 mg of **N-(1-((4,4-di-fluorocyclohexyl)methyl)-1H-indol-5-yl)acrylamide** as a white solid, yield 50%.

### Example 23 N-(1-((tetrahydro-2H-pyran-4-yl)methyl)-1H-indol-5-yl)acrylamide

**1-((tetrahydro-2H-pyran-4-yl)methyl)-1H-indol-5-amine** (138 mg, 599 µmol) were solubilized in dichloromethane (6 ml) with Et₃N (126 µl, 719 µmol) cooled down to 0°C before adding acryloyl chloride (58 µl, 718 µmol). Stirring was continued for 45 min at 0°C. The reaction mixture was diluted with dichloromethane, extracted with water, dried over Na₂SO₄, evaporated at reduced pressure and purified by column chromatography (silica gel, gradient DCM/MeOH 100/0 to 95/5) to obtain 106 mg of **N-(1-((tetrahydro-2H-pyran-4-yl)methyl)-1H-indol-5-yl)acrylamide** as a white solid, yield 62%.

### Example 24 N-(1-((4-(trifluoromethyl)cyclohexyl)methyl)-1H-indol-5-yl)acrylamide

**1-((4-(trifluoromethyl)cyclohexyl)methyl)-1H-indol-5-amine** (114 mg, 384 µmol) were solubilized in dichloromethane (4 ml) with Et₃N (81 µl, 461 µmol) cooled down to 0°C before adding acryloyl chloride (38 µl, 461 µmol). Stirring was continued for 45 min at 0°C. The reaction mixture was diluted with dichloromethane, extracted with water, dried over Na₂SO₄, evaporated at reduced pressure and purified by column chromatography (silica gel, gradient cyclohexane/AcOEt 100/0 to 50/50) to obtain 70 mg of **N-(1-((4-(trifluoromethyl)cyclohexyl)methyl)-1H-indol-5-yl)acrylamide** as a pink solid, yield 52%.

### Example 25 N-(1-((1,1-difluorospiro[2.3]hexan-5-yl)methyl)-1H-indol-5-yl)acrylamide

**1-((1,1-difluorospiro[2.3]hexan-5-yl)methyl)-1H-indol-5-amine** (110 mg, 419 µmol) were solubilized in dichloromethane (2 ml) with Et₃N (70 µl, 503 µmol) cooled down to 0°C before adding acryloyl chloride (38 µl, 461 µmol). Stirring was continued for 30 min at 0°C. The reaction mixture was diluted with CH₂Cl₂, extracted with water, dried over Na₂SO₄, evaporated at reduced pressure and purified by column chromatography (silica gel, gradient heptane/AcOEt) to obtain after trituration in diethylether 75mg of **N-(1-((1,1-difluorospiro[2.3]hexan-5-yl)methyl)-1H-indol-5-yl)acrylamide** as solid, yield 57%.

### Example 26 N-(1-((3-fluorocyclopentyl)methyl)-1H-indol-5-yl)acrylamide

**1-((3-fluorocyclopentyl)methyl)-1H-indol-5-amine** (130 mg, 560 µmol) were solubilized in dichloromethane (2 ml) with Et₃N (94 µl, 672 µmol) cooled down to 0°C before adding acryloyl chloride (52 µl, 616 µmol). Stirring was continued for 30 min at 0°C. The reaction mixture was diluted with dichloromethane, extracted with water, dried over Na₂SO₄, evaporated at reduced pressure and purified by column chromatography (silica gel, gradient heptane/AcOEt 95/5 to 60/40) to obtain after trituration in diethylether 72 mg of **N-(1-((3-fluorocyclopentyl)methyl)-1H-indol-5-yl)acrylamide** as solid, yield 45%.

### Example 27 N-(1-(cyclohexylmethyl)-1H-indol-5-yl)acrylamide

**1-(cyclohexylmethyl)-1H-indol-5-amine** (260 mg, 1.14 mmol) were solubilized in dichloromethane (10 ml) with Et₃N (218.76 µl, 1.25 mmol), cooled down to 0°C before adding acryloyl chloride (97 µl, 1.20 mmol). Stirring was continued for 45 min at 0°C. The reaction mixture was diluted with CH₂Cl₂, extracted with water, dried over Na₂SO₄, evaporated at reduced pressure and purified by column chromatography (silica gel, gradient heptane/AcOEt 100/0 to 50/50) to obtain after trituration in AcOEt 77 mg of **N-(1-(cyclohexylmethyl)-1H-indol-5-yl)acrylamide** as a solid, yield 24%.

### Example 28 N-(1-((3-(trifluoromethyl)cyclobutyl)methyl)-1H-indol-5-yl)acrylamide

**1-((3-(trifluoromethyl)cyclobutyl)methyl)-1H-indol-5-amine** (138 mg, 514 µmol) were solubilized in dichloromethane (2 ml) with Et₃N (86 µl, 617 µmol), cooled down to 0°C before adding acryloyl chloride (48 µl, 566 µmol). Stirring was continued for 45 min at 0°C. The reaction mixture was diluted with dichloromethane, extracted with water, dried over Na₂SO₄, evaporated at reduced pressure and purified by column chromatography (silica gel, gradient heptane/AcOEt 95/5 to 60/40) to obtain after trituration in diethylether 47 mg of N-**(1-((3-(trifluoromethyl)cyclobutyl)methyl)-1**H-indol-5-yl)acrylamide as solid, yield 28%.

### Example 29 N-(1-((3,3-difluorocyclopentyl)methyl)-1H-indol-5-yl)acrylamide

**1-((3,3-difluorocyclopentyl)methyl)-1H-indol-5-amine** (67 mg, 268 µmol) were solubilized in dichloromethane (2 ml) with Et₃N (45 µl, 321 µmol), cooled down to 0°C before adding acryloyl chloride (25 µl, 295 µmol). Stirring was continued for 1h at 0°C. The reaction mixture was diluted with dichloromethane, extracted with water, dried over Na₂SO₄, evaporated at reduced pressure and purified by column chromatography (silica gel, gradient heptane/AcOEt 95/5 to 60/40) to obtain 34 mg of **N-(1-((3,3-difluorocyclopentyl)methyl)-1H-indol-5-yl)acrylamide** as a solid, yield 42%.

Diaryl-derivatives were introduced either via the already described alkylation in step 1 of the general scheme (see example 30) or as a variation of the general synthetic scheme where a Suzuki coupling-step allows the synthesis of the corresponding diaryl-erivatives for non-commercially available building-blocks (see example 31) after the first step of the general scheme.

### Example 30 N-(1-([1,1'-biphenyl]-4-ylmethyl)-1H-indol-5-yl)acrylamide

**1-([1,1'-biphenyl]-4-ylmethyl)-1H-indol-5-amine** (106 mg, 355 µmol) were solubilized in dichloromethane (5 ml) with Et₃N (74 µl, 533 µmol), cooled down to 0°C before adding acryloyl chloride (40 mg, 426 µmol). Stirring was continued for 2h at RT. The reaction mixture was diluted with dichloromethane, extracted with water and an aqueous saturated solution of NaCl, dried over Na₂SO₄, evaporated at reduced pressure and purified by column chromatography (silica gel, gradient heptane/AcOEt 100/0 to 70/30) to obtain 56 mg of **N-(1-([1,1'-biphenyl]-4-ylmethyl)-1H-indol-5-yl)acrylamide** as a white solid, yield 42%.

### Example 31 N-(1-((3'-(trifluoromethyl)-[1,1'-biphenyl]-4-ylmethyl)-1H-indol-5-yl)-acrylamide

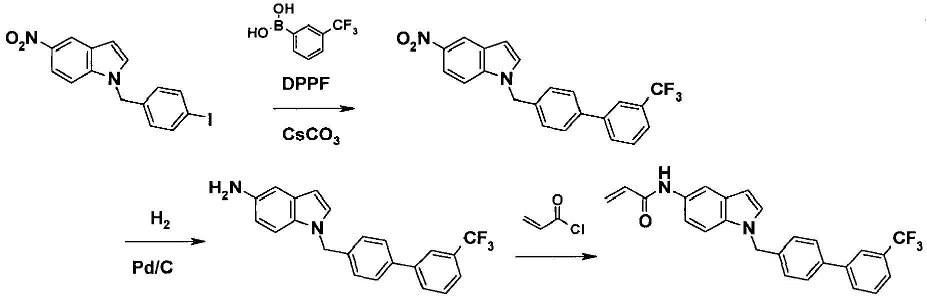

### 1-(4-iodobenzyl)-5-nitro-1H-indole was synthesized as described in example 1 (step1). 5-nitro-1-[[4-[3-(trifluoromethyl)phenyl]phenyl]methyl]indole

To a suspension of **1-(4-iodobenzyl)-5-nitro-1H-indole** (300 mg, 793 µmol) in 1,4-dioxane (5 ml) and water (1 ml) were added 3-(trifluoromethyl)phenylboronic acid (158 mg, 793 µmol), cesium carbonate (516 mg, 1,59 mmol), DPPF (22 mg, 40 µmol) and PdCl₂(PPh₃)₂ (28mg, 40 µmol), the mixture was heated to 80°C (all components were solubilized at this temperature), stirring was continued for 4h at 80°C. The reaction mixture was diluted with AcOEt, extracted with water and an aqueous saturated solution of NaCl, dried over Na₂SO₄, evaporated at reduced pressure and purified by column chromatography (silica gel, gradient heptane/AcOEt 100/0 to 75/25) to obtain 253 mg of **5-nitro-1-[[4-[3-(trifluoromethyl)phenyl]-phenyl]methyl]indole** as a yellow resin, yield 90%.

### 1-([1,1'-biphenyl]-4-ylmethyl)-1H-indol-5-amine was synthesized and purified as described in example 1 (step2).

### N-(1-([1,1'-biphenyl]-4-ylmethyl)-1H-indol-5-yl)acrylamide

**1-((3'-(trifluoromethyl)-[1,1'-biphenyl]-4-yl)methyl)-1H-indol-5-amine** (188 mg, 513 µmol) were solubilized in dichloromethane (5 ml) with Et₃N (86 µl, 616 µmol), cooled down to 0°C before adding acryloyl chloride (58 mg, 616 µmol). Stirring was continued for 2h at RT. The reaction mixture was diluted with dichloromethane, extracted with water and an aqueous saturated solution of NaCl, dried over Na₂SO₄, evaporated at reduced pressure and purified by column chromatography (silica gel, gradient heptane/AcOEt 100/0 to 50/50) to obtain 31 mg of **N-(1-((3'-(trifluoromethyl)-[1,1'-biphenyl]4-yl)methyl)-1H-indol-5-yl)acrylamide (example 31)** as a white solid, yield 14%.

### Example 32 N-(1-((4'-(trifluoromethyl)-[1,1'-biphenyl]-4-yl)methyl)-1H-indot-5-yl)-acrylamide

**1-((4'-(trifluoromethyl)-[1,1'-biphenyl]-4-yl)methyl)-1H-indol-5-amine** (158 mg, 431 µmol) were solubilized in dichloromethane (5 ml) with Et₃N (72 µl, 518 µmol), cooled down to 0°C before adding acryloyl chloride (49 mg, 518 µmol). Stirring was continued for 1h at RT. The reaction mixture was diluted with dichloromethane, extracted with water, dried over Na₂SO₄, evaporated at reduced pressure and purified by column chromatography (silica gel, gradient DCM/MeOH 100/0 to 95/5) to obtain after trituration in dichloromethane 60 mg of **N-(1-((4'-(trifluoromethyl)-[1,1'-biphenyl]-4-yl)methyl)-1H-indol-5-yl)acrylamide** as a white solid, yield 30%.

An alternative for step 1 in scheme 2 is represented in the following example. A Mitsunobu reaction approach was used with primary or secondary alcohols as starting materials. Step 2 and 3 were realized in the same manner as illustrated in preceding schemes.

### Example 33 Trans-N-[1-[4-(trifluoromethyl)cyclohexyl]indol-5-yl]prop-2-enamide and Example 34 Cis-N-[-1-[4-(trifluoromethyl)cyclohexyl]indol-5-yl]prop-2-enamide

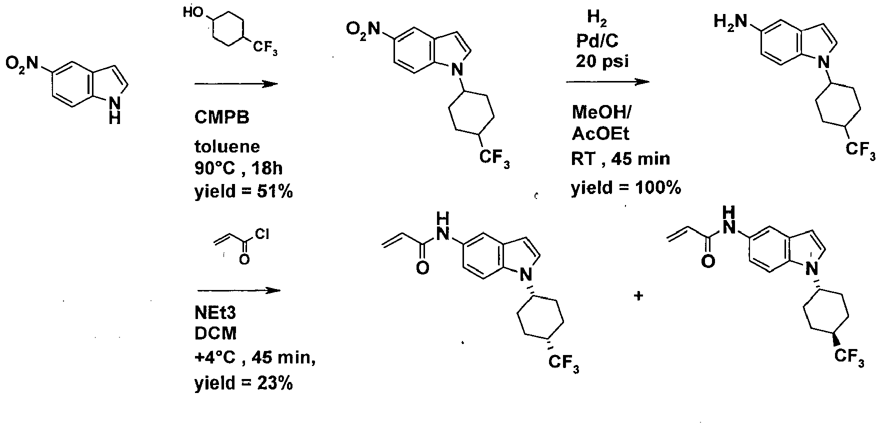

### 5-nitro-1-[4-(trifluoromethyl)cyclohexyl]indole

**4-(trifluoromethyl)cyclohexanol** (341 µl, 2.42 mmol) was introduced under nitrogen in toluene (30 ml), and 5-nitroindole (200 mg, 1.21 mmol) and CMPB (988 µl, 3.63 mmol) were added sequentially. The resulting mixture was heated at 90°C for 18h and was stirred at RT for the following 24h and then diluted by AcOEt and washed two times by water and two times by an aqueous saturated solution of NaCl. The organic layer was dried over solid Na₂SO₄ and the solvent was removed under reduced pressure to afford after column chromatography purification (silica gel, eluant DCM/MeOH 100/0 to 99/1) 194mg of **5-nitro-1-[4-(trifluoromethyl)cyclohexyl]indole** as a solid, yield 51%.

### 1-(4-(trifluoromethyl)cyclohexyl)-1H-indol-5-amine

**5-nitro-1-(4-(trifluoromethyl)cyclohexyl)-1H-indole** (194 mg, 621 µmol) were solubilized in 5 ml of methanol and 5ml of AcOEt, Pd/C 10% (50%wet) (66 mg) was added and the mixture was hydrogenated in a Parr apparatus at RT under 20psi hydrogen pressure for 45min. After filtration and evaporation at reduced pressure, 180 mg of **1-(4-(trifluoromethyl)cyclohexyl)-1H-indol-5-amine** were obtained and used without further purification, yield 100%.

### N-[1-[4-(trifluoromethyl)cyclohexyl]indol-5-yl]prop-2-enamide

**1-(4-(trifluoromethyl)cyclohexyl)-1H-indol-5-amine** (175 mg, 620 µmol) were solubilized in dichloromethane (2 ml) with Et₃N (104 µl, 744 µmol), cooled down to 0°C before adding acryloyl chloride (57 µl, 682 µmol). Stirring was continued for 45min at RT. The reaction mixture was diluted with dichloromethane, extracted with water, dried over Na₂SO₄, evaporated at reduced pressure and purified by column chromatography (silica gel, gradient heptane/AcOEt 95/5 to 60/40) to obtain 23 mg of ***cis*-N-[1-[4-(trifluoromethyl)cyclohexyl]indol-5-yl]prop-2-enamide** (example 33) as a white solid (yield 7%) and after further trituration in diethylether 52 mg of ***trans*-N-[1-[4-(trifluoromethyl)cyclohexyl]indol-5-yl]prop-2-enamide** (example 34) as a white powder (yield 16%).

### Example 35 N-(1-((1-(4-(trifluoromethyl)phenyl)piperidin-4-yl)methyl]-1H-indol-5-yl)-acrylamide

**1-((1-(4-(trifluoromethyl)phenyl)piperidin-4-yl)methyl)-1H-indol-5-amine** (280 mg, 900 µmol) were solubilized in dichloromethane (7ml) with Et₃N (220 µl, 1.50 mmol), cooled down to 0°C before adding acryloyl chloride (73 µl, 1.1 mmol). Stirring was continued for 1h at RT. The reaction mixture was diluted with dichloromethane, extracted with water, dried over Na₂SO₄, evaporated at reduced pressure and purified by column chromatography (silica gel, gradient heptane/AcOEt 100/0 to 70/30) to obtain 30 mg of a foam (80% purity) further purified via reversed phase column chromatography to obtain 12 mg of **N-(1-((1-(4-(trifluoromethyl)phenyl)piperidin-4-yl)methy1)-1H-indol-5-yl)-acrylamide** as an amorphous solid, yield 3.7%.

### Example 36 N-(1-(2-(methyl(2,2,2-trifluoroethyl)amino)ethyl)-1H-indol-5-yl)acrylamide

**1-(2-(methyl(2,2,2-trifluoroethyl)amino)ethyl)-1H-indol-5-amine** (150 mg, 550 µmol) were solubilized in dichloromethane (2 ml) with Et₃N (120 µl, 830 µmol), cooled down to 0°C before adding acryloyl chloride (56 µl, 660 µmol). Stirring was continued for 2h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain after trituration in pentane86 mg of **N-(1-(2-(methyl(2,2,2-trifluoroethyl)amino)ethyl)-1H-indol-5-yl)acrylamide** as a pale beige solid, yield 48%.

### Example 37 N-(1-(2-(2,2,2-trifluoroethoxy)ethyl)-1H-indol-5-yl)acrylamide

**1-(2-(2,2,2-trifluoroethoxy)ethyl)-1H-indol-5-amine** (224 mg, 0.87 mmol) were solubilized in dichloro-methane (5 ml) with Et₃N (180 µl, 1.3 mmol), cooled down to 0°C before adding acryloyl chloride (88 µl, 1.04 mmol). Stirring was continued for 4h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain after trituration in diethylether 64 mg of **N-(1-(2-(2,2,2-trifluoroethoxy)ethyl)-1H-indol-5-yl)acrylamide** as a white solid, yield 23%.

### Example 38 N-(1-(1-(2,2,2-trifluoroethyl)piperidin-4-yl)-1H-indo)-5-yl)acrylamide

**1-(1-(2,2,2-trifluoroethyl)piperidin-4-yl)-1H-indol-5-amine** (73 mg, 246 µmol) were solubilized in dichloromethane (3ml) with Et₃N (72 µl, 491 µmol), cooled down to 0°C before adding acryloyl chloride (24 µl, 295 µmol). Stirring was continued for 1h at RT. The reaction mixture was diluted with dichloromethane, extracted with water, dried over Na₂SO₄, evaporated at reduced pressure and purified by column chromatography (silica gel, gradient heptane/AcOEt 100/0 to 70/30) to obtain 20 mg of **N-(1-(1-(2,2,2-trifluoro-ethyl)-piperidin-4-yl)-1H-indol-5-yl)acrylamide** as an amorphous solid, yield 23%.

### Example 39 N-(1-(4,4-difluorocyclohexyl)-1H-indol-5-yl)acrylamide

**1-(4,4-difluorocyclohexyl)-1H-indol-5-amine** (134 mg, 540 µmol) were solubilized in dichloromethane (3 ml) with Et₃N (90 µl, 640 µmol), cooled down to 0°C before adding acryloyl chloride (88 µl, 1.04 mmol). Stirring was continued for 15 min at 0°C. The reaction mixture was diluted with dichloromethane, extracted with water, dried over Na₂SO₄, evaporated at reduced pressure and purified by column chromatography (silica gel, gradient heptane/AcOEt 95/5 to 60/40) to obtain 42 mg of **N-(1-(2-(2,2,2-trifluoroethoxy)ethyl)-1H-indol-5-yl)acrylamide** as a powder, yield 68%.

### Example 40 N-(1-(1-(4-(trifluoromethyl)phenyl)piperidin-4-yl)-1H-indol-5-yl)acrylamide

**1-(1-(4-(trifluoromethyl)phenyl)piperidin-4-yl)-1H-indol-5-amine** (60 mg, 167 µmol) was solubilized in dichloromethane (2 ml) with Et₃N (28 µl, 200 µmol), cooled down to 0°C before adding acryloyl chloride (16 µl, 184 µmol). Stirring was continued for 30 min at 0°C. The reaction mixture was diluted with dichloromethane, extracted with water and an aqueous saturated solution of NaCl, dried over Na₂SO₄, evaporated at reduced pressure and purified by column chromatography (silica gel, gradient heptane/AcOEt 95/5 to 60/40) to obtain 32 mg of **N-(1-(1-(4-(trifluoromethyl)phenyl)piperidin-4-yl)-1H-indol-5-yl)-acrylamide** as a white powder, yield 51%.

Another variation of the general approach is represented in the following example 41 where a halogenated alcohol is introduced in the first step via the already described Mitsunobu-reaction followed by further substitution by a secondary amine.

### Example 41 N-(1-(3-(3,3-difluoropyrrolidin-1-yl)propyl)-1H-indol-5-yl)acrylamide

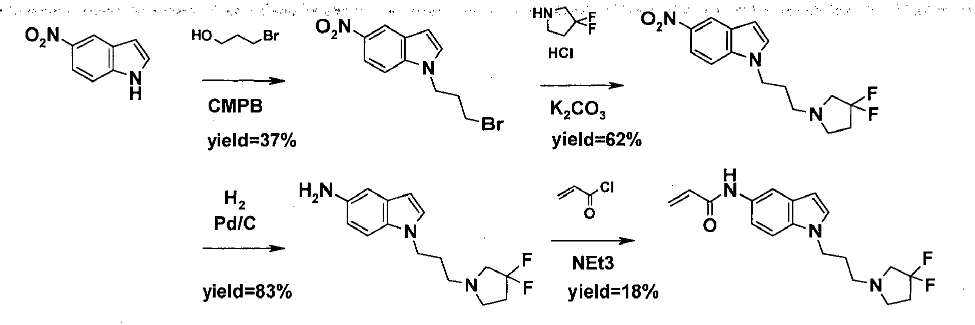

### 1-(3-bromopropyl)-5-nitro-1H-indole

**5-nitroindole** (1g, 6.04 mmol) were solubilized in toluene (100 ml) before adding 3-bromo-1-propanol (1.77 g, 12.09 mmol) et CMPB (4.61 g, 18.13 mmol). The mixture was heated at 100°C for 2h, cooled to RT, diluted with AcOEt, washed with water and an aqueous saturated solution of NaCl, dried over sodium sulfate and concentrated under reduced pressure. Purification by column chromatography on silica gel (eluant heptane/AcOEt 100/0 to 50/50) provided 2.2 g of a yellow solid. Yield 37%.

### 1-(3-(3,3-difluoropyrrolidin-1-yl)propyl)-5-nitro-1H-indole

**3,3-difluoropyrrolidine hydrochloride** (136 mg, 918 µmol), potassium carbonate (225 mg, 1.62 mmol) and 1-(3-bromopropyl)-5-nitro-1H-indole (200 mg, 706 µmol) were solubilized in acetonitrile (5 ml). The mixture was heated at 70°C overnight, then cooled to RT, poured into water and extracted with AcOEt. The organic layer was dried over sodium sulfate and concentrated under reduced pressure. The residue was purified by column chromatography on silica gel (eluant heptane/AcOEt 100/0 to 50/50) to obtain 136mg of 1-(3-(3,3-difluoropyrrolidin-1-yl)propyl)-5-nitro-1H-indole as a yellow oil. Yield 62%.

### 1-(3-(3,3-difluoropyrrolidin-1-yl)propyl)-1H-indol-5-amine

**1-(3-(3,3-difluoropyrrolidin-1-yl)propyl)-5-nitro-1H-indole** (136 mg, 440 µmol) were solubilized in MeOH (10ml). Pd/C 10% (50% wet) (47 mg, 22 µmol) was added and the mixture was hydrogenated in a Parr apparatus at RT under 20psi hydrogen pressure for 2h. After filtration and evaporation under reduced pressure, 100 mg of a pinky coloured oil were obtained and used without further purification in the next step. Yield 81%.

### N-(1-(3-(3,3-difluoropyrrolidin-1-yl)propyl)-1H-indol-5-yl)acrylamide (Example 41)

1-(3-(3,3-difluoropyrrolidin-1-yl)propyl)-1H-indol-5-amine (100 mg, 358 µmol) were solubilized in dichloromethane (2 ml) with Et₃N (75 µl, 537 µmol), cooled down to 0°C before adding acryloyl chloride (36 µl, 430 µmol). Stirring was continued for 2h at RT. The reaction mixture was diluted with dichloromethane, extracted with water, dried over Na₂SO₄, evaporated at reduced pressure and purified by column chromatography (silica gel, gradient heptane/AcOEt 100/0 to 50/50) to obtain after trituration in diethylether 22 mg of N-(1-(3-(3,3-difluoropyrrolidin-1-yl)propyl)-1H-indol-5-yl)acrylamide as a solid, yield 18%.

The following example presents an alternative for the reduction of the nitro-group (step 2 of scheme 2) in order to tolerate functional groups sensitive to catalytic hydrogenation.

### Example 42 N-(1-(4-(trimethylsilyl)benzyl)-1H-indol-5-vl)acrvlamide

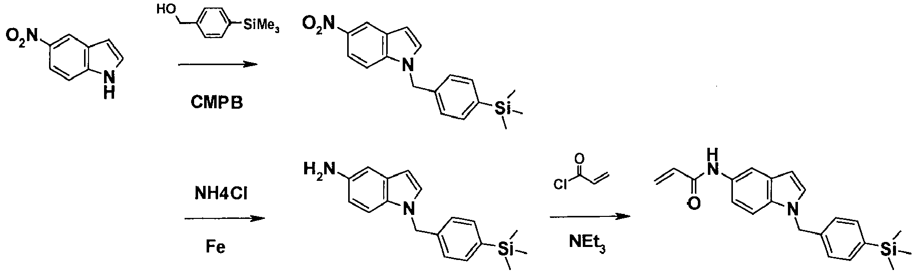

### 5-nitro-1-(4-(trimethylsilyl)benzyl)-1H-indole

**4-(trimethylsilyl)phenyl)methanol** (556 mg, 3.08mmol) was introduced under nitrogen in toluene (15 ml). 5-nitroindole (250 mg, 1.54 mmol) and CMPB (1.3 ml, 4.63 mmol) were added sequentially. The resulting mixture was heated at 90°C for 18h and then diluted by AcOEt and washed two times with water and two times with an aqueous saturated solution of NaCl. The organic layer was dried over solid Na₂SO₄ and the solvent was removed under reduced pressure. After column chromatography purification (silica gel, eluant DCM/MeOH 100/0 to 99/1) 194 mg of **5-nitro-1-(4-(trimethylsilyl)benzyl)-1H-indole** was obtained as a pale yellow solid, yield 51%.

### 1-(4-(trimethylsilyl)benzyl)-1H-indol-5-amine

To a solution of **5-nitro-1-(4-(trimethylsilyl)benzyl)-1H-indole** (200mg, 0.62 mmol) in 4.5ml of ethanol and 1.5ml of water were added ammonium chloride (87 mg, 1.54mmol) and iron powder (87 mg, 1.54mml). The pale-yellow solution darkened rapidly and was heated under reflux for 2h. After cooling down to RT 3ml of water were added. The resulting gel was filtered through a Whatman filter-pad and rinsed with AcOEt. The aqueous layer was separated and extracted twice with AcOEt. The combined organic layers were washed with water and an aqueous saturated solution of NaCl, dried over Na₂SO₄ and evaporated under reduced pressure to obtain 110 mg of a gum used without further purification for the next step, raw yield 60%.

### N-(1-(4-(trimethylsilyl)benzyl)-1H-indol-5-yl)acrylamide (example 42)

**N-(1-(4-(trimethylsilyl)benzyl)-1H-indol-5-amine** (105 mg, 36 µmol) were solubilized in dichloromethane (6 ml) with Et₃N (99 µl, 713 µmol) cooled down to 0°C before adding acryloyl chloride (36 µl, 430 µmol). Stirring was continued for 18h at RT. The reaction mixture was diluted with dichloromethane, extracted with water, dried over Na₂SO₄, evaporated at reduced pressure and purified by column chromatography (silica gel, gradient heptane/AcOEt 100/0 to 70/30) to obtain 50 mg of **N-(1-(4-(trimethylsilyl)-benzyl)-1H-indol-5-yl)acrylamide** as a white solid, yield 36%.

Another sequence is illustrated in the following example where the first step of the general scheme is realized via an Ullmann-coupling by using iodo-aryl or -hetero-aryl derivatives:

### Example 43 N-(1-(3-(trifluoromethyl)phenyl)-1H-indol-5-yl)acrylamide

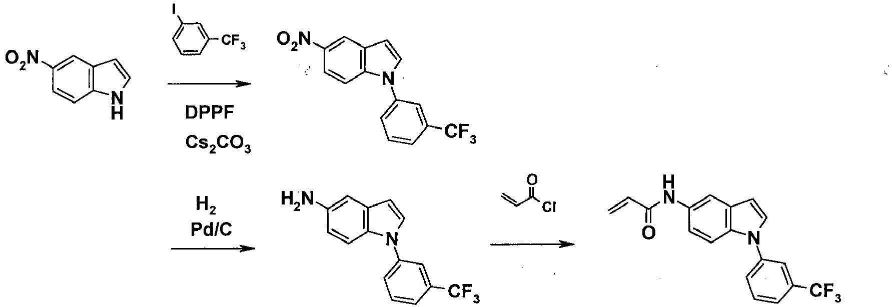

### 5-nitro-1-(3-(trifluoromethyl)phenyl)-1H-indole

As described in patent application US 2016/318864, 5-nitroindole (500 mg, 3.02 mmol), 1-iodo-3-(trifluoromethyl)benzene (536 µl, 3.02 mmol), N,N'-dimethylethylenediamine (331 µl, 3.05 mmol), copper(I) iodide (587 mg, 3.08 mmol) and potassium carbonate (852 mg, 6.16 mmol) were added in DMSO (5 ml) and heated at 140°C for 6h. AcOEt is added to the mixture and washed with water and with a saturated aqueous solution of NaCl. The organic layer is dried over MgSO₄. After filtration and evaporation of the solvent at reduced pressure, 850mg of **5-nitro-1-(3-(trifluoromethyl)phenyl)-1H-indole** were obtained and used without further purification, yield 92%.

### 1-(3-(trifluoromethyl)phenyl)-1H-indol-5-amine

**5-nitro-1-(3-(trifluoromethyl)phenyl)-1H-indole** (0.85 g, 2.78 mmol) were solubilized in 20 ml of MeOH and 5ml of AcOEt, Pd/C 10% (50% wet) (30 mg) was added and the mixture was hydrogenated in a Parr apparatus at RT under 30psi hydrogen pressure for 2h. After filtration and evaporation at reduced pressure 714 mg of **1-(3-(trifluoro-methyl)phenyl)-1H-indol-5-amine** were obtained as a brown oil and used without further purification, yield 93%.

**1-(3-(trifluoromethyl)phenyl)-1H-indol-5-amine** (200 mg, 724 µmol) obtained in the step described above were solubilized in dichloromethane (2 ml) with Et₃N (121 µl, 869 µmol), cooled down to 0°C before adding acryloyl chloride (82 mg, 869 µmol). Stirring was continued for 1h30 at RT. The reaction mixture was diluted with dichloromethane, extracted with water, dried over Na₂SO₄, evaporated at reduced pressure and purified by column chromatography (silica gel, gradient heptane/AcOEt 95/5 to 60/40) to obtain after trituration in diethylether, 102 mg of **N-(1-(3-(trifluoromethyl)phenyl)-1H-indol-5-yl)acryl-amide (example 43)** as a white solid, yield 42%.

The following compounds were synthesized in a similar manner as example 43 by using the appropriate indoles and primary or secondary alcohols as starting materials known to those skilled in the art.

### Example 44 N-(1-(4-(trifluoromethyl)phenyl)-1H-indol-5-yl)acrylamide

**1-(4-(trifluoromethyl)phenyl)-1H-indol-5-amine** (200 mg, 724 µmol) were solubilized in dichloromethane (2 ml) with Et₃N (121 µl, 869 µmol), cooled down to 0°C before adding acryloyl chloride (82 mg, 869 µmol). Stirring was continued for 3h at RT. The reaction mixture was diluted with dichloromethane, extracted with water, dried over Na₂SO₄, evaporated at reduced pressure and purified by column chromatography (silica gel, gradient heptane/AcOEt 95/5 to 60/40) to obtain after trituration in diethylether, 105 mg of **N-(1-(4-(trifluoromethyl)phenyl)-1H-indol-5-yl)acrylamide** as a white solid, yield 42%.

### Example 45 N-(1-(6-(trifluoromethyl)pyridin-3-yl)-1H-indol-5-yl)acrylamide

**1-(6-(trifluoromethyl)pyridin-3-yl)-1H-indol-5-amine** (173 mg 603 µmol) were solubilized in dichloromethane (5 ml) with N,N-diisopropylethylamine (160 µl, 909 µmol), cooled down to 0°C before adding acryloyl chloride (50 µl, 617 µmol). Stirring was continued overnight at RT. The reaction mixture was diluted with dichloromethane, extracted with water, dried over Na₂SO₄, evaporated at reduced pressure and purified by column chromatography (silica gel, gradient cyclohexane/AcOEt 100/0 to 80/20) to obtain after trituration in diisopropylether, 67 mg of **N-(1-(6-(trifluoro-methyl)-pyridin-3-yl)-1H-indol-5-yl)acrylamide** as a white solid, yield 32%.

### Example 46 N-(1-(5-(trifluoromethyl)pyridin-2-yl)-1H-indol-5-yl)acrylamide

**1-(5-(trifluoromethyl)pyridin-2-yl)-1H-indol-5-amine** (167 mg, 572 µmol) were solubilized in dichloromethane (5 ml) with N,N-diisopropylethylamine (160 µl, 909 µmol), cooled down to 0°C before adding acryloyl chloride (50 µl, 617 µmol). Stirring was continued overnight at RT. The reaction mixture was diluted with dichloromethane, extracted with water, dried over Na₂SO₄, evaporated at reduced pressure and purified by column chromatography (silica gel, gradient cyclohexane/AcOEt 100/0 to 80/20) to obtain after trituration in diisopropylether, 67 mg of **N-(1-(5-(trifluoromethyl)pyridin-2-yl)-1H-indol-5-yl)acryl-amideas** a white solid, yield 35%.

### Example 47 N-(1-(pyridin-3-yl)-1H-indol-5-yl)acrylamide

**1-(pyridin-3-yl)-1H-indol-5-amine** (224 mg, 867 µmol) were solubilized in dichloro-methane (5 ml) with N,N-diisopropylethylamine (230 µl, 1.31 mmol), cooled down to 0°C before adding acryloyl chloride (80 µl, 988 µmol). Stirring was continued overnight at RT. The reaction mixture was diluted with dichloromethane, extracted with water, dried over Na₂SO₄, evaporated at reduced pressure and purified by column chromatography (silica gel, gradient cyclohexane/AcOEt 100/0 to 60/40) to obtain after trituration in diisopropylether 136 mg of **N-(1-(pyridin-3-yl)-1H-indol-5-yl)acrylamide** as a white solid, yield 57%.

### Example 48 N-(1-(4-fluorophenyl)-1H-indol-5-yl)acrylamide

**1-(4-fluorophenyl)-1H-indol-5-amine** (183 mg, 809 µmol) were solubilized in dichloromethane (4 ml) with Et₃N (169 µl, 1.21 mmol), cooled down to 0°C before adding acryloyl chloride (92 mg, 971 µmol). Stirring was continued for 4h at RT. The reaction mixture was diluted with dichloromethane, extracted with water, dried over Na₂SO₄, evaporated at reduced pressure and purified by column chromatography (silica gel, gradient heptane/AcOEt 100/0 to 70/30) to obtain 38 mg of **N-(1-(4-fluorophenyl)-1H-indol-5-yl)-acrylamide** as a white solid , yield 17%.

### Example 49 N-(1-(3,4-difluorophenyl)-1H-indol-5-vl)acrvlamide

**1-(3,4-difluorophenyl)-1H-indol-5-amine** (310 mg, 1.27 mmol) were solubilized in dichloromethane (4 ml) with Et₃N (265 µl, 1.90 mmol), cooled down to 0°C before adding acryloyl chloride (144 mg, 1.52 mmol). Stirring was continued for 4h at RT. The reaction mixture was diluted with dichloromethane, extracted with water, dried over Na₂SO₄, evaporated at reduced pressure and purified by column chromatography (silica gel, gradient heptane/AcOEt 100/0 to 70/30) to obtain 5 mg of **N-(1-(3,4-difluorophenyl)-1H-indol-5-yl)acrylamide** as a pale pink solid, yield 2%.

### Example 50 N-(1-(3-(methylsulfonyl)phenyl-1H-indol-5-vl)acrvlamide

**1-(3-(methylsulfonyl)phenyl)-1H-indol-5-amine** (255 mg, 891 µmol) were solubilized in dichloromethane (5 ml) with Et₃N (186 µl, 1.34 mmol), cooled down to 0°C before adding acryloyl chloride (101 mg, 1.07 mmol). Stirring was continued for 4h at RT. The reaction mixture was diluted with dichloromethane, extracted with water, dried over Na₂SO₄, evaporated at reduced pressure and purified by column chromatography (silica gel, gradient heptane/AcOEt 100/0 to 70/30) to obtain 115 mg of **N-(1-(3-(methylsulfonyl)-phenyl)-1H-indol-5-yl)acrylamide** as a white solid, yield 34%.

### Example 51 N-1-(4-cyanophenyl)-1H-indol-5-yl)crylamide

**4-(5-amino-1H-indol-1-yl)benzonitrile** (32 mg, 137 µmol) were solubilized in dichloromethane (3 ml) with Et₃N (29 µl, 206 µmol), cooled down to 0°C before adding acryloyl chloride (16 mg, 165 µmol). Stirring was continued for 2h at RT. The reaction mixture was diluted with dichloromethane, extracted with water, dried over Na₂SO₄, evaporated at reduced pressure and purified by column chromatography (silica gel, gradient heptane/AcOEt 100/0 to 60/40) to obtain 8 mg of **N-(1-(4-cyanophenyl)-1H-indol-5-yl)-acrylamide** as a white solid, yield 20%.

The table herein below illustrates the chemical structures and physical properties of a number of compounds of formula (I).

In the table:
- the proton magnetic resonance spectra (¹H NMR), as described below, are recorded at 400 MHz, 500 MHz or 600 MHz in DMSO-d₆, using the DMSO-d₆ peak as reference. The chemical shifts δ are expressed in parts per million (ppm). The signals observed are expressed as follows: s = singlet; d = doublet; t = triplet; m = multiplet or br s = broad singlet; br m = broad multiplet
- the LCMS characteristics, as described below, successively indicated the high-performance liquid chromatography analytical method used and detailed below (A and B), the [M+H]⁺ peak identified by mass spectrometry and the retention time (Tr) of the compound, expressed in minutes.

### Method A

Column: Acquity UPLC/SQD CORTECS C18+ 2.1x50mm 1.6µm (Waters)
Gradient: MeCN/H₂O with 0.1%. FA from 2 to 100% during 10min, 1ml/min

### Method B

Column: Acquity UPLC/SDS BEH C18 2.1x50mm 1.7µm (Waters)
Gradient: MeCN (+0.035% FA)/H₂O (+0.05% FA) from 5% to 95% in 3min, 0.9ml/min

| example N° | IUPAC name | NMR Description | [M+H] | Tr (min) | Analytical method |
|---|---|---|---|---|---|
| 1 | N-[1-[[3-(trifluoromethyl) phenyl]methyl]-indol-5-yl]prop-2-enamide | 1H NMR (400 MHz, DMSO-d6) δ ppm 5.51 (s, 2 H) 5.70 (dd, J=10.04, 2.13 Hz, 1 H) 6.22 (dd, J=16.94, 2.13 Hz, 1 H) 6.38 - 6.54 (m, 2 H) 7.28 (dd, J=8.78, 2.01 Hz, 1 H) 7.37 - 7.46 (m, 2 H) 7.49 - 7.58 (m, 3 H) 7.59 - 7.64 (m, 1 H) 8.00 (d, J=1.76 Hz, 1 H) 9.96 (s, 1 H) | 345 | 1.23 | A |
| 2 | N-methyl-N-(1-(3-(trifluoromethyl) benzyl)-1H-indol-5-yl)acrylamide | 1H NMR (400 MHz, DMSO-d6) δ ppm 3.24 (s, 3 H) 5.46 (dd, J=10.16, 2.38 Hz, 1 H) 5.56 (s, 2 H) 5.97 (dd, J=17.06, 10.16 Hz,1 H) 6.10 (dd, J=17.06, 2.38 Hz, 1 H) 6.54 (d, J=3.01 Hz, 1 H) 7.00 (dd, J=8.53, 2.01 Hz, 1 H) 7.45 (d, J=2.01 Hz, 1 H) 7.49 (d, J=7.62 Hz, 1 H) 7.54 - 7.60 (m, 2 H) 7.62 - 7.71 (m, 3 H) | 359 | 1.43 | A |
| 3 | N-(1-(5,5,5-trifluoropentyl)-1H-indol-5-yl)acrylamide | 1H NMR (400 MHz, DMSO-d6) δ ppm 1.37 - 1.48 (m, 2 H) 1.81 (quin, J=7.34 Hz, 2 H) 2.18 - 2.35 (m, 2 H) 4.18 (t, J=6.90 Hz, 2 H) 5.70 (dd, J=10.12, 2.01 Hz, 1 H) 6.22 (dd, J=17.07, 2.01 Hz, 1 H) 6.39 (d, J=2.76 Hz, 1 H) 6.45 (dd, J=17.07, 10.12 Hz, 1 H) 7.31 (dd, J=8.78, 2.01 Hz, 1 H) 7.35 (d, J=2.76 Hz, 1 H) 7.44 (d, J=8.78 Hz, 1 H) 7.97 (d, J=2.01 Hz, 1 H) 9.98 (s, 1 H) | 311 | 1.25 | A |
| 4 | N-(1-(1-(3-(trifluoromethyl) phenyl)ethyl)-1 H-indol-5-yl)acrylamide | 1H NMR (400 MHz, DMSO-d6) δ ppm 1.90 (d, J=7.03 Hz, 3 H) 5.69 (dd, J=10.04, 2.01 Hz, 1 H) 5.92 (q, J=7.03 Hz, 1 H) 6.21 (dd, J=16.94, 2.01 Hz, 1 H) 6.43 (dd, J=16.94, 10.04 Hz,1 H) 6.52 (d, J=3.26 Hz, 1 H) 7.24 (dd, J=8.91, 1.88 Hz, 1 H) 7.36 (d, J=8.91 Hz, 1 H) 7.46 - 7.65 (m, 4 H) 7.71 (d, J=3.26 Hz, 1 H) 7.99 (d, J=1.88 Hz, 1 H) 9.94 (s, 1 H) | 359 | 1.38 | A |
| 5 | N-(1-(3-(pentafluoro-lambda6-sulfanyl)benzyl)-1 H-indol-5-yl)acrylamide | 1H NMR (400 MHz, DMSO-d6) δ ppm 5.53 (s, 2 H) 5.69 (dd, J= 10.26, 2.28 Hz, 1 H) 6.22 (dd, J=17.07, 2.28 Hz, 1 H) 6.38 - 6.56 (m, 2 H) 7.28 (dd, J=8.78, 1.76 Hz, 1 H) 7.37 (d, J=7.53 Hz, 1 H) 7.42 (d, J=8.78 Hz, 1 H) 7.51 - 7.62 (m, 2 H) 7.74-7.81 (m, 2 H) 8.00 (d, J=1.76 Hz, 1 H) 9.96 (s, 1 H) | 403 | 1.37 | A |
| 6 | N-(1-(4-(pentafluoro-lambda6-sulfanyl)benzyl)-1 H-indol-5-yl)acrylamide | 1H NMR (400 MHz, DMSO-d6) δ ppm 5.52 (s, 2 H) 5.70 (dd, J=10.16, 2.13 Hz, 1 H) 6.22 (dd, J=17.07, 2.13 Hz, 1 H) 6.38 - 6.55 (m, 2 H) 7.23 - 7.39 (m, 4 H) 7.50 (d, J=3.01 Hz, 1 H) 7.84 (d, J=8.87 Hz, 2 H) 8.01 (d, J=1.76 Hz, 1 H) 9.96 (s, 1 H) | 403 | 1.36 | A |
| 7 | N-(1-(4-(trifluoro-methoxy)benzyl) -1H-indol-5-yl)acrylamide | 1H NMR (400 MHz, DMSO-d6) δ ppm 5.44 (s, 2 H) 5.70 (dd, J=10.16, 2.13 Hz, 1 H) 6.22 (dd, J=17.07, 2.13 Hz, 1 H) 6.39 - 6.51 (m, 2 H) 7.25 - 7.33 (m, 5 H) 7.38 (d, J=8.78 Hz, 1 H) 7.49 (d, J=3.26 Hz, 1 H) 8.00 (d, J=1.76 Hz, 1 H) 9.95 (s, 1 H) | 361 | 1.32 | A |
| 8 | N-(1-(3-(trifluoro-methoxy)benzyl) -1H-indol-5-yl)acrylamide | 1H NMR (400 MHz, DMSO-d6) δ ppm 5.46 (s, 2 H) 5.70 (dd, J=10.28, 2.23 Hz, 1 H) 6.22 (dd, J=16.94, 2.23 Hz, 1 H) 6.40 - 6.51 (m, 2 H) 7.14 - 7.19 (m, 2 H) 7.21 - 7.31 (m, 2 H) 7.36 - 7.47 (m, 2 H) 7.51 (d, J=3.26 Hz, 1 H) 8.00 (d, J=1.76 Hz, 1 H) 9.95 (s, 1 H) | 361 | 1.30 | A |
| 9 | N-(1-(3-iodobenzyl)-1 H-indol-5-yl)-acrylamide | 1H NMR (400 MHz, DMSO-d6) δ ppm 5.37 (s, 2 H) 5.70 (dd, J=10.16, 2.13 Hz, 1 H) 6.22 (dd, J=16.94, 2.13 Hz, 1 H) 6.40 - 6.52 (m, 2 H) 7.10 (t, J=7.71 Hz, 1 H) 7.17 (d, J=8.03, 1 H) 7.28 (dd, J=8.78, 2.01 Hz, 1 H) 7.38 (d, J=8.78 Hz, 1 H) 7.48 (d, J=3.01 Hz, 1 H) 7.54 - 7.67 (m, 2 H) 8.00 (d, J=2.01 Hz, 1 H) 9.95 (s, 1 H) | 403 | 1.36 | A |
| 10 | N-(1-(2-fluoro-5-(trifluoromethyl)-benzyl)-1H-indol-5-yl)-acrylamide | 1H NMR (400 MHz, DMSO-d6) δ ppm 5.54 (s, 2 H) 5.70 (dd, J=10.16, 2.13 Hz, 1 H) 6.23 (dd, J=16.94, 2.13 Hz, 1 H) 6.40 - 6.52 (m, 2 H) 7.31 (dd, J=8.78, 2.01 Hz, 1 H) 7.38 - 7.54 (m, 4 H) 7.70 - 7.80 (m, 1 H) 8.00 (d, J=2.01 Hz, 1 H) 9.97 (s, 1 H) | 363 | 4.23 | A |
| 11 | N-(3-methyl-1-(3-(trifluoromethyl)-benzyl)-1H-indol-5-yl)acrylamide | 1H NMR (500 MHz, DMSO-d6) δ ppm 2.23 (d, J=0.73 Hz, 3 H) 5.43 (s, 2 H) 5.70 (dd, J=10.03, 1.96 Hz, 1 H) 6.22 (dd, J=16.99, 1.96 Hz, 1 H) 6.44 (dd, J=16.99, 10.03 Hz, 1 H) 7.25 - 7.32 (m, 2 H) 7.34 - 7.43 (m, 2 H) 7.53 (t, J=7.70 Hz, 1 H) 7.58 - 7.63 (m, 2 H) 7.94 (d, J=1.71 Hz, 1 H) 9.97 (s, 1 H) | 359 | 1.39 | A |
| 12 | N-(1-(3-methoxybenzyl)-1 H-indol-5-yl)-acrylamide | 1H NMR (400 MHz, DMSO-d6) δ ppm 3.69 (s, 3 H) 5.35 (s, 2 H) 5.69 (dd, J=10.28, 2.13 Hz, 1 H) 6.22 (dd, J=16.94, 2.13 Hz, 1 H) 6.40 - 6.50 (m, 2 H) 6.69 - 6.85 (m, 3 H) 7.20 (t, J=7.91 Hz, 1 H) 7.27 (dd, J=8.78, 2.01 Hz, 1 H) 7.37 (d, J=8.78 Hz, 1 H) 7.47 (d, J=3.01 Hz, 1 H) 7.99 (d, J=2.01 Hz, 1 H) 9.94 (s, 1 H) | 307 | 1.24 | A |
| 13 | N-(2-methyl-1-(3-(trifluoromethyl)-benzyl)-1H-indol-5-yl)-acrylamide | 1H NMR (400 MHz, DMSO-d6) δ ppm 2.33 (s, 3 H) 5.50 (s, 2 H) 5.69 (dd, J=10.16, 2.13 Hz, 1 H) 6.22 (dd, J=16.94, 2.13 Hz, 1 H) 6.29 (s, 1 H) 6.44 (dd, J=16.94, 10.16 Hz, 1 H) 7.14 (d, J=7.53 Hz, 1 H) 7.21 (dd, J=8.78, 1.76 Hz, 1 H) 7,30 (d, J=8.72 Hz, 1 H) 7.38 (s, 1 H) 7.52 (t, J=8.72 Hz, 1 H) 7.60 (d, J=8.72 Hz, 1 H) 7.91 (d, J=1.76 Hz, 1 H) 9.92 (s, 1 H) | 359 | 1.41 | A |
| 14 | N-(3-cyano-1-(3-(trifluoromethyl) benzyl)-1H-indol-5-yl)-acrylamide | 1H NMR (400 MHz, DMSO-d6) δ ppm 5.60 (s, 2 H) 5.76 (dd, J= 10.16, 2.010, 1 H) 6.26 (dd, J=17.07, 2.01 Hz, 1 H) 6.44 (dd, J=17.07, 10.16 Hz, 1 H) 7.38 - 7.82 (m, 6 H) 8.20 (d, J=1.51 Hz, 1 H) 8.47 (s, 1 H) 10.20 (s, 1 H) | 370 | 1.32 | A |
| 15 | N-(1-(3-cyclopropyl-benzyl)-1H-indol-5-yl)-acrylamide | 1H NMR (400 MHz, DMSO-d6) δ ppm 0.55 - 0.63 (m, 2 H) 0.86 - 0.95 (m, 2 H) 1.79 - 1.88 (m, 1 H) 5.32 (s, 2 H) 5.69 (dd, J=10.04, 2.13 Hz, 1 H) 6.22 (dd, J=16.94, 2.13 Hz, 1 H) 6.40 - 6.50 (m, 2 H) 6.86 - 6.94 (m, 2 H) 6.96 (s, 1 H) 7.15 (t, J=7.69 Hz, 1 H) 7.27 (dd, J=8.78, 1.76 Hz, 1 H) 7.38 (d, J=8.78 Hz, 1 H) 7.46 (d, J=3.01 Hz, 1 H) 7.98 (d, J=1.76 Hz, 1 H) 9.94 (s, 1 H) | 317 | 1.43 | A |
| 16 | N-(2,3-dimethyl-1-(3-(trifluoromethyl)-benzyl)-1H-indol-5-yl)-acrylamide | 1H NMR (400 MHz, DMSO-d6) δ ppm 2.19 (s, 3 H) 2.26 (s, 3 H) 5.47 (s, 2 H) 5.69 (dd, J=10.16, 2.13 Hz, 1 H) 6.22 (dd, J=16.94, 2.13 Hz, 1 H) 6.45 (dd, J=16.94, 10.16 Hz, 1 H) 7.11 (d, J=7.78 Hz, 1 H) 7.23 (dd, J=8.76, 1.72 Hz, 1 H) 7.21 (d, J=8.76 Hz, 1 H) 7.42 (s, 1 H) 7.50 (t, J=7.78 Hz, 1 H) 7.59 (t, J=7.78 Hz, 1 H) 7.88 (d, J=1.72 Hz, 1 H) 9.94 (s, 1 H) | 373 | 1.57 | A |
| 17 | N-(1-(3-methylbenzyl)-1 H-indol-5-yl)-acrylamide | 1H NMR (500 MHz, DMSO-d6) δ ppm 2.24 (s, 3 H) 5.34 (s, 2 H) 5.69 (dd, J=10.15, 2.08 Hz, 1 H) 6.22 (dd, J=16.87, 2.08 Hz, 1 H) 6.40 - 6.50 (m, 2 H) 6.96 (d, J=7.58 Hz, 1 H) 7.01 - 7.07 (m, 2 H) 7.18 (t, J=7.58 Hz, 1 H) 7.26 (dd, J=8.80, 1.96 Hz, 1 H) 7.37 (d, J=8.80 Hz, 1 H) 7.45 (d, J=2.93 Hz, 1 H) 7.98 (d, J=1.96 Hz, 1 H) 9.94 (s, 1 H) | 291 | 1.48 | A |
| 18 | N-(1-Benzyl-1H-indol-5-yl)-acrylamide | 1H NMR (600 MHz, DMSO-d6) δ ppm 5.35 (s, 2 H) 5.70 (dd, J=10.04, 2.13 Hz, 1 H) 6.22 (dd, J=16.94, 2.13 Hz, 1 H) 6.38 - 6.54 (m, 2 H) 7.15 - 7.20 (m, 1 H) 7.21 - 7.31 (m, 5 H) 7.32- 7.40 (m, 1 H) 7.45 - 7.55 (m, 1 H) 8.00 (s, 1 H) 10.00 (s, 1 H) | 276 | 1.66 | B |
| 19 | N-(1-(3-(trifluoromethyl) benzyl)-1 H-indol-4-yl)-acrylamide | 1H NMR (400 MHz, DMSO-d6) δ ppm 5.53 (s, 2 H) 5.75 (dd, J=10.04, 2.01 Hz, 1 H) 6.28 (dd, J=16.81, 2.01 Hz, 1 H) 6.71 (dd, J=16.81, 10.04 Hz, 1 H) 6.82 (d, J=3.01 Hz, 1 H) 7.07 (t, J=8.10 Hz, 1 H) 7.24 (d, J=8.28 Hz, 1 H) 7.42 (d, J=7.78 Hz, 1 H) 7.49 - 7.68 (m, 4 H) 7.75 (br d, J=7.53 Hz, 1 H) 9.78 (s, 1 H) | 345 | 1.35 | A |
| 20 | (E)-4-(dimethylamino)-N-(1-(3-(trifluorom ethyl) benzyl)-1 H-indol-4-yl)but-2-enamide | 1H NMR (400 MHz, DMSO-d6) δ ppm 2.61 (br s, 6 H) 3.58- 3.75 (m, 2 H) 5.54 (s, 2 H) 6.63 - 6.91 (m, 3 H) 7.07 (t, J=7.91 Hz, 1 H) 7.25 (d, J=8.28 Hz, 1 H) 7.43 (br d, J=7.78 Hz, 1 H) 7.50 - 7.66 (m, 4 H) 7.75 (br d, J=7.53 Hz, 1 H) 9.91 (s, 1 H) | 402 | 0.84 | A |
| 21 | N-(1-(3-(trifluoromethyl) benzyl)-1 H-indol-6-yl)-acrylamide | 1H NMR (400 MHz, DMSO-d6) δ ppm 5.49 (s, 2 H) 5.70 (dd, J=10.04, 2.01 Hz, 1 H) 6.21 (dd, J=16.94, 2.01 Hz, 1 H) 6.38 - 6.52 (m, 2 H) 7.18 (dd, J=8.53, 1.76 Hz, 1 H) 7.36 (d, J=7.53 Hz, 1 H) 7.46 - 7.68 (m, 5 H) 7.96 (s, 1 H) 10.05 (s, 1 H) | 345 | 1.39 | A |
| 22 | N-(1-((4,4-difluorocyclohex yl)methyl)-1H-indol-5-yl)-acrylamide | 1H NMR (400 MHz, DMSO-d6) δ ppm 1.20 - 1.35 (m, 2 H) 1.51 - 1.83 (m, 4 H) 1.89 - 2.08 (m, 3 H) 4.07 (d, J=7.28 Hz, 2 H) 5.70 (dd, J=10.16, 2.13 Hz, 1 H) 6.23 (dd, J=16.94, 2.13 Hz, 1 H) 6.39 (d, J=3.01 Hz, 1 H) 6.45 (dd, J=16.94, 10.16 Hz, 1 H) 7.25 - 7.36 (m, 2 H) 7.45 (d, J=8.78 Hz, 1 H) 7.95 (d, J=3.01 Hz, 1 H) 9.94 (s, 1 H) | 319 | 1.25 | A |
| 23 | N-(1-((tetrahydro-2H-pyran-4-yl)methyl)-1H-indol-5-yl)-acrylamide | 1H NMR (400 MHz, DMSO-d6) δ ppm 1.19 - 1.43 (m, 4 H) 1.94 - 2.11 (m, 1 H) 3.20 (td, J=11.54, 2.26 Hz, 2 H) 3.81 (br dd, J=11.29, 2.51 Hz, 2 H) 4.04 (d, J=7.28 Hz, 2 H) 5.70 (dd, J=10.04, 2.01 Hz, 1 H) 6.22 (dd, J=16.94, 2.01 Hz, 1 H) 6.38 (d, J=3.01 Hz, 1 H) 6.45 (dd, J=16.94, 10.04 Hz, 1 H) 7.22 - 7.36 (m, 2 H) 7.45 (d, J=8.78 Hz, 1 H) 7.95 (d, J=1.76 Hz, 1 H) 9.94 (s, 1 H) | 285 | 1.00 | A |
| 24 | N-(1-((4-(trifluoromethyl)-cyclohexyl)-methyl)-1H-indol-5-yl)-acrylamide | 1H NMR (400 MHz, DMSO-d6) mixture of isomers 60/40 δ ppm 0.91 - 1.93 (m, 9 H) 2.09 - 2.24 (m, 1 H) 4.00 (d, J=7.28 Hz, 1.2 H) 4.14 (d, J=7.78 Hz, 0.8 H) 5.70 (dd, J=10.04, 2.01 Hz, 1 H) 6.23 (dd, J=16.94, 2.01 Hz, 1 H) 6.38 (d, J=3.01 Hz, 1 H) 6.45 (dd, J=17.07, 10.04 Hz, 1 H) 7.22 - 7.37 (m, 2 H) 7.41 (d, J=8.78 Hz, 1 H) 7.91 - 8.01 (m, 1 H) 9.94 (s, 1 H) | 351 | 1.46 | A |
| 25 | N-(1-((1,1-difluorospiro[2.3] hexan-5-yl)-methyl)-1 H-indol-5-yl)-acrylamide | 1H NMR (400 MHz, DMSO-d6)) mixture of isomers 65/35 δ ppm 1.32 - 1.43 (m, 2 H) 1.94 - 2.25 (m, 4 H) 2.78 - 3.02 (m, 1 H) 4.22 (d, J=7.53 Hz, 0.70 H) 4.27 (d, J=7.28 Hz, 1.3 H) 5.70 (dd, J=10.04, 2.01 Hz, 1 H) 6.23 (dd, J=16.94, 2.01 Hz, 1 H) 6.39 (d, J=3.01 Hz, 1 H) 6.45 (dd, J=16.94, 10.04 Hz, 1 H) 7.28 - 7.50 (m, 3 H) 7.96 (d, J=1.51 Hz, 1 H) 9.94 (s, 1 H) | 317 | 1.36 | A |
| 26 | N-( 1-((3-fluoro-cyclopentyl)-methyl)-1 H-indol-5-yl)-acrylamide | 1H NMR (400 MHz, DMSO-d6) δ ppm 1.27 - 2.12 (m, 6 H) 2.57 - 2.65 (m, 1 H) 4.11 (d, J=7.28 Hz, 2 H) 5,15 (dt, J=54,89, 5,16 Hz, 1 H) 5.70 (dd, J=10.16, 2.13 Hz, 1 H) 6.23 (dd, J=16.94, 2.13 Hz, 1 H) 6.39 (d, J=3.01 Hz, 1 H) 6.45 (dd, J=16.94, 10.16 Hz, 1 H) 7.31 (dd, J=8.91, 1.88 Hz, 1 H) 7.36 (d, J=3.01 Hz, 1 H) 7.45 (d, J=8.91 Hz, 1 H) 7.96 (d, J=1.88 Hz, 1 H) 9.94 (s, 1 H) | 287 | 3.92 | A |
| 27 | N-(1-(cyclohexylmethyl)-1H-indol-5-yl)-acrylamide | 1H NMR (400 MHz, DMSO-d6) δ ppm 0.88 - 1.22 (m, 5 H) 1.49 (br d, J=12.55 Hz, 2 H) 1.55 - 1.70 (m, 3 H) 1.72 - 1.87 (m, 1 H) 3.97 (d, J=7.28 Hz, 2 H) 5.69 (dd, J=10.16, 2.13 Hz, 1 H) 6.22 (dd, J=16.94, 2.13 Hz, 1 H) 6.37 (d, J=3.01 Hz, 1 H) 6.45 (dd, J=16.94, 10.16 Hz, 1 H) 7.27 - 7.33 (m, 2 H) 7.40 (d, J=8.78 Hz, 1 H) 7.95 (d, J=1.51 Hz, 1 H) 9.93 (s, 1 H) | 283 | 1.47 | A |
| 28 | N-( 1-((3-(trifluoromethyl)cyclobutyl)methyl)-1 H-indol-5-yl)-acrylamide | 1H NMR (400 MHz, DMSO-d6)) mixture of isomers 70/30 δ ppm 1.81 - 1.93 (m, 1 H) 1.97 - 2.20 (m, 2 H) 2.64 - 2.88 (m, 1 H) 3.92 - 3.13 (m, 1 H) 3.19 - 3.27 (m, 1 H) 4.15 (d, J=6.78 Hz, 0.6 H) 4.27 (d, J=7.53 Hz, 1.4 H) 5.69 (dd, J=10.04, 2.01 Hz, 1 H) 6.23 (dd, J=17.07, 2.01 Hz, 1 H) 6.38 (d, J=3.26 Hz, 1 H) 6.45 (dd, J=17.07, 10.04 Hz, 1 H) 7.27 - 7.43 (m, 2 H) 7.48 (d, J=8.78 Hz, 1 H) 7.96 (d, J=1.25 Hz, 1 H) 9.94 (s, 1 H) | 323 | 1.39 | A |
| 29 | N-(1-((3,3-difluorocyclopentyl)methyl)-1 H-indol-5-yl)-acrylamide | 1H NMR (400 MHz, DMSO-d6) δ ppm 1,42 - 1.62 (m, 1 H) 1.69 - 2.28 (m, 5 H) 2.57 - 2.65 (m, 1 H) 4.01 - 4,25 (m, 2 H) 5.70 (dd, J=10.04, 2.01 Hz, 1 H) 6.23 (dd, J=16.94, 2.01 Hz, 1 H) 6.40 (d, J=2.51 Hz, 1 H) 6.45 (dd, J=16.94, 10.04 Hz, 1 H) 7.31 (dd, J=8.78, 2.01 Hz, 1 H) 7.37 (d, J=2.51 Hz, 1 H) 7.46 (d, J=8.78 Hz, 1 H) 7.97 (d, J=2.01 Hz, 1 H) 9.95 (s, 1 H) | 305 | 1.38 | A |
| 30 | N-(1-([1,1'-biphenyl]-4-yl-methyl)-1H-indol-5-yl)-acrylamide | 1H NMR (400 MHz, DMSO-d6) δ ppm 5.44 (s, 2 H) 5.70 (dd, J=10.16, 2.13 Hz, 1 H) 6.22 (dd, J=16.94, 2.13 Hz, 1 H) 6.38 - 6.54 (m, 2 H) 7.23 - 7.37 (m, 4 H) 7.38 - 7.47 (m, 3 H) 7.51 (d, J=3.01 Hz, 1 H) 7.55 - 7.65 (m, 4 H) 8.00 (d, J=1.66 Hz, 1 H) 9.95 (s, 1 H) | 353 | 1.46 | A |
| 31 | N-(1-((3'-(trifluoromethyl)-[1,1'-biphenyl]-4-yl)-methyl)-1H-indol-5-yl)-acrylamide | 1H NMR (400 MHz, DMSO-d6) δ ppm 5.46 (s, 2 H) 5.70 (dd, J=10.04, 2.01 Hz, 1 H) 6.22 (dd, J=17.07, 2.01 Hz, 1 H) 6.40 - 6.54 (m, 2 H) 7.25 - 7.34 (m, 3 H) 7.41 (d, J=8.78 Hz, 1 H) 7.52 (d, J=3.26 Hz, 1 H) 7.64 - 7.73 (m, 4 H) 7.89 - 7.96 (m, 2 H) 8.00 (d, J=1.76 Hz, 1 H) 9.95 (s, 1 H) | 421 | 1.53 | A |
| 32 | N-(1-((4'-(trifluoromethyl)-[1,1'-biphenyl]-4-yl)methyl)-1H-indol-5-yl)-acrylamide | 1H NMR (400 MHz, DMSO-d6) δ ppm 5.46 (s, 2 H) 5.69 (dd, J=10.24, 2.11 Hz, 1 H) 6.22 (dd, J=16.94, 2.11 Hz, 1 H) 6.40 - 6.53 (m, 2 H) 7.25 - 7.34 (m, 3 H) 7.42 (d, J=8.78 Hz, 1 H) 7.52 (d, J=3.01 Hz, 1 H) 7.67 (d, J=8.30, 2 H) 7.78 (d, J=8.53 Hz, 2 H) 7.84 (d, J=8.53 Hz, 2 H) 8.00 (d, J=1.76 Hz, 1 H) 9.95 (s, 1 H) | 421 | 1.54 | A |
| 33 | N-(1-((trans)-4-(trifluoromethyl)-cyclohexyl)-1H-indol-5-yl)-acrylamide | 1H NMR (400 MHz, DMSO-d6) δ ppm 1.52 - 1.73 (m, 2 H) 1.81 - 1.97 (m, 2 H) 1.98 - 2.11 (m, 4 H) 2.38 - 2.48 (m, 1 H) 4.30 - 4.50 (m, 1 H) 5.70 (dd, J=2.13, 10.06 Hz, 1 H) 6.23 (dd, J=16.94, 2.13 Hz, 1 H) 6.39 - 6.53 (m, 2 H) 7.31 (dd, J=8.78, 2.01 Hz, 1 H) 7.40 (d, J=3.26 Hz, 1 H) 7.52 (d, J=8.78 Hz, 1 H) 7.96 (d, J=2.01 Hz, 1 H) 9.94 (s, 1 H) | 337 | 1.46 | A |
| 34 | N-(1-((cis)-4-(trifluoromethyl)-cyclohexyl)-1 H-indol-5-yl)-acrylamide | 1H NMR (400 MHz, DMSO-d6) δ ppm 1.82 - 2.11 (m, 8 H) 2.55 - 2.65 (m, 1 H) 4.40 - 4.58 (m, 1 H) 5.70 (dd, J=10.04, 2.01 Hz, 1 H) 6.23 (dd, J=16.94, 2.01 Hz, 1 H) 6.37 - 6.52 (m, 2 H) 7.32 (dd, J=8.91, 1.88 Hz, 1 H) 7.40 - 7.51 (m, 2 H) 7.97 (d, J=1.88 Hz, 1 H) 9.95 (s, 1 H) | 337 | 1.44 | A |
| 35 | N-(1-((1-(4-(trifluorom ethyl)-phenyl)piperidin-4-yl)methyl)-1 H-indol-5-yl)-acrylamide | 1H NMR (400 MHz, DMSO-d6) δ ppm 1.21 - 1.39 (m, 2 H) 1.52 (br d, J=10.79 Hz, 2H) 1.99 - 2.14 (m, 1 H) 2.69 - 2.83 (m, 2 H) 3.86 (br d, J=12.80 Hz, 2 H) 4.07 (d, J=7.28 Hz, 2 H) 5.70 (dd, J=10.04, 2.01 Hz, 1 H) 6.23 (dd, J=16.94, 2.01 Hz, 1 H) 6.39 (d, J=1.76 Hz, 1 H) 6.46 (dd, J=16.94, 10.04 Hz, 1 H) 7.02 (d, J=8.78 Hz, 2 H) 7.26 - 7.37 (m, 2 H) 7.41 - 7.53 (m, 3 H) 7.96 (d, J=1.76 Hz, 1 H) 9.94 (s, 1 H) | 428 | 1.58 | A |
| 36 | N-(1-(2-(methyl(2,2,2-trifluoroethyl)-amino)ethyl)-1H-indol-5-yl)-acrylamide | 1H NMR (400 MHz, DMSO-d6) δ ppm 2.40 (s, 3 H) 2.92 (t, J=6.78 Hz, 2 H) 3.16 - 3.28 (m, 2 H) 4.23 (t, J=6.78 Hz, 2 H) 5.70 (dd, J=10.04, 2.01 Hz, 1 H) 6.23 (dd, J=16.94, 2.01 Hz, 1 H) 6.38 (d, J=2.76 Hz, 1 H) 6.45 (dd, J=16.94, 10.04 Hz, 1 H) 7.31 (dd, J=8.78, 1.76 Hz, 1 H) 7.35 (d, J=2.76 Hz, 1 H) 7.43 (d, J=8.78 Hz, 1 H) 7.96 (d, J=1.76 Hz, 1 H) 9.94 (s, 1 H) | 326 | 1.24 | A |
| 37 | N-(1-(2-(2,2,2-trifluoroethoxy)-ethyl)-1 H-indol-5-yl)acrylamide | 1H NMR (400 MHz, DMSO-d6) δ ppm 3.90 (t, J=5.40 Hz, 2 H) 3.95 - 4.08 (m, 2 H) 4.35 (t, J=5.40 Hz, 2 H) 5.70 (dd, J=10.16, 2.01 Hz, 1 H) 6.23 (dd, J=17.07, 2.01 Hz, 1 H) 6.39 (d, J=3.01 Hz, 1 H) 6.45 (dd, J=17.07, 10.16 Hz, 1 H) 7.26 - 7.36 (m, 2 H) 7.44 (d, J=8.78 Hz, 1 H) 7.96 (d, J=1.76 Hz, 1 H) 9.94 (s, 1 H) | 313 | 1.17 | A |
| 38 | N-(1-(1-(2,2,2-trifluoroethyl)-piperidin-4-yl)-1H-indol-5-yl)-acrylamide | 1H NMR (400 MHz, DMSO-d6) δ ppm 1.84 - 2.07 (m, 4 H) 2.58 - 2.71 (m, 2 H) 3.06 (br d, J=11.80 Hz, 2 H) 3.19 - 3.29 (m, 2 H) 4.27 - 4.40 (m, 1 H) 5.69 (dd, J=10.07 - 2.13, 1 H) 6.23 (dd, J=16.94, 2.13 Hz, 1 H) 6.38 - 6.53 (m, 2 H) 7.31 (dd, J=8.91, 1.88 Hz, 1 H) 7.44 - 7.54 (m, 2 H) 7.96 (d, J=1.76 Hz, 1 H) 9.94 (s, 1 H) | 352 | 1.12 | A |
| 39 | N-(1-(4,4-difluorocyclo-hexyl)-1H-indol-5-yl)acrylamide | 1H NMR (400 MHz, DMSO-d6) δ ppm 1.91 - 2.29 (m, 8 H) 4.51 - 4.65 (m, 1 H) 5.70 (dd, J=10.04, 2.01 Hz, 1 H) 6.23 (dd, J=16.94, 2.01 Hz, 1 H) 6.40 - 6.52 (m, 2 H) 7.33 (dd, J=8.91, 1.88 Hz, 1 H) 7.44 - 7.54 (m, 2 H) 7.97 (d, J=1.88 Hz, 1 H) 9.95 (s, 1 H) | 305 | 1.38 | A |
| 40 | N-(1-(1-(4-(trifluoromethyl) phenyl)piperidin-4-yl)-1 H-indol-5-yl)acrylamide | 1H NMR (400 MHz, DMSO-d6) δ ppm 1.94 - 2.14 (m, 4 H) 3.02 - 3.15 (m, 2 H) 4.06 (br d, J=12.80 Hz, 2 H) 4.53 - 4.68 (m, 1 H) 5.70 (dd, J= 10.07, 2.13 Hz, 1 H) 6.23 (dd, J=16.94, 2.13 Hz, 1 H) 6.38 - 6.51 (m, 2 H) 7.14 (d, J=8.78 Hz, 2 H) 7.33 (dd, J=8.78, 1.76 Hz, 1 H) 7.47 (d, J=3.26 Hz, 1 H) 7.49 - 7.57 (m, 3 H) 7.97 (d, J=1.76 Hz, 1 H) 9.95 (s, 1 H) | 414 | 1.53 | A |
| 41 | N-(1-(3-(3,3-difluoropyrrolidin -1-yl)propyl)-1H-indol-5-yl)-acrylamide | 1H NMR (400 MHz, DMSO-d6) δ ppm 1.89 (quin, J=6.84 Hz, 2 H) 2.17 - 2.36 (m, 4 H) 2.64 (t, J=7.03 Hz, 2 H) 2.83 (t, J=13.43 Hz, 2 H) 4.18 (t, J=6.78 Hz, 2 H) 5.69 (dd, J=10.08, 2.13 Hz, 1 H) 6.23 (dd, J=16.94, 2.13 Hz, 1 H) 6.39 (d, J=2.76 Hz, 1 H) 6.45 (dd, J=16.94, 10.08 Hz, 1 H) 7.27 - 7.36 (m, 2 H) 7.41 (d, J=8.69 Hz, 1 H) 7.97 (d, J=1.76 Hz, 1 H) 9.94 (s, 1 H) | 334 | 1.05 | A |
| 42 | N-(1-(4-(trimethylsilyl)-benzyl)-1H-indol-5-yl)-acrylamide | 1H NMR (400 MHz, DMSO-d6) δ ppm 0.14 - 0.24 (m, 9 H) 5.37 (s, 2 H) 5.69 (dd, J=10.16, 2.13 Hz, 1 H) 6.22 (dd, J=17.07, 2.13 Hz, 1 H) 6.38 - 6.50 (m, 2 H) 7.16 (d, J=8.03 Hz, 2 H) 7.26 (dd, J=8.78, 2.01 Hz, 1 H) 7.37 (d, J=8.78 Hz, 1 H) 7.41 - 7.50 (m, 3 H) 7.98 (d, J=2.01 Hz, 1 H) 9.94 (s, 1 H) | 349 | 1.55 | A |
| 43 | N-(1-(3-(trifluoromethyl) phenyl)-1H-indol-5-yl)-acrylamide | 1H NMR (400 MHz, DMSO-d6) δ ppm 5.73 (dd, J=10.15, 1.94 Hz,1 H) 6.25 (dd, J=16.94, 1.94 Hz, 1 H) 6.47 (dd, J=16.94 10.15 Hz, 1 H) 6.73 (d, J=3.26 Hz, 1 H) 7.40 (dd, J=8.78, 2.01 Hz, 1 H) 7.55 (d, J=8.78 Hz, 1 H) 7.70 - 7.86 (m, 3 H) 7.89 - 8.02 (m, 2 H) 8.15 (d, J=2.01 Hz, 1 H) 10.13 (s, 1 H) | 331 | 1.37 | A |
| 44 | N-(1-(4-(trifluoromethyl) phenyl)-1H-indol-5-yl)-acrylamide | 1H NMR (400 MHz, DMSO-d6) δ ppm 5.73 (dd, J=10.13, 2.01 Hz, 1 H) 6.26 (dd, J=17.07, 2.01 Hz, 1 H) 6.4- (dd, J=17.07, 10.13 Hz, 1 H) 6.76 (d, J=2.76 Hz, 1 H) 7.42 (dd, J=8.78, 2.01 Hz, 1 H) 7.65 (d, J=8.78 Hz, 1 H) 7.77 (d, J=2.76 Hz, 1 H) 7.85 (d, J=8.36 Hz, 2 H) 7.93 (d, J=8.36 Hz, 2 H) 8.14 (d, J=2.01 Hz, 1 H) 10.14 (s, 1 H) | 331 | 1.39 | A |
| 45 | N-(1-(6-(trifluoromethyl) pyridin-3-yl)-1H-indol-5-yl)-acrylamide | 1H NMR (400 MHz, DMSO-d6) δ ppm 5.74 (dd, J=10.13, 2.01 Hz, 1 H) 6.26 (dd, J=17.07, 2.01 Hz, 1 H) 6.47 (dd, J= 17.07, 10.13 Hz, 1 H) 6.81 (d, J=3.51 Hz, 1 H) 7.45 (dd, J=8.91, 1.88 Hz, 1 H) 7.70 (d, J=8.91 Hz, 1 H) 7.85 (d, J=3.51 Hz, 1 H) 8.09 (d, J=8.28 Hz, 1 H) 8.15 (d, J=1.88 Hz, 1 H) 8.36 (dd, J=8.28, 2.26 Hz, 1 H) 9.09 (d, J=2.26 Hz, 1 H) 10.12 (s, 1 H) | 332 | 1.19 | A |
| 46 | N-(1-(5-(trifluoromethyl) pyridin-2-yl)-1H-indol-5-yl)-acrylamide | 1H NMR (400 MHz, DMSO-d6) δ ppm 5.74 (dd, J=10.04, 2.01 Hz, 1 H) 6.27 (dd, J=16.81, 2.01 Hz, 1 H) 6.48 (dd, J=16.81, 10.04 Hz, 1 H) 6.83 (d, J=3.51 Hz, 1 H) 7.46 (dd, J=9.03, 2.01 Hz, 1 H) 7.99 (d, J=8.78 Hz, 1 H) 8.12 - 8.20 (m, 2 H) 8.32 (dd, J=8.78, 2.38 Hz, 1 H) 8.52 (d, J=9.03 Hz, 1 H) 8.93 (d, J=2.38 Hz, 1 H) 10.15 (s, 1 H) | 332 | 1.31 | A |
| 47 | N-( 1-(pyridin-3-yl)-1 H-indol-5-yl)acrylamide | 1H NMR (400 MHz, DMSO-d6) δ ppm 5.73 (dd, J=10.16, 1.88 Hz, 1 H) 6.26 (dd, J=16.81, 1.88 Hz, 1 H) 6.47 (dd, J=16.81, 10.16 Hz, 1 H) 6.74 (d, J=3.01 Hz, 1 H) 7.41 (dd, J=8.91, 1.88 Hz, 1 H) 7.55 (d, J=8.91 Hz, 1 H) 7.58 - 7.64 (m, 1 H) 7.73 (d, J=3.01 Hz, 1 H) 8.04 - 8.10 (m, 1 H) 8.13 (d, J=1.88 Hz, 1 H) 8.59 (dd, J=4.77, 1.25 Hz, 1 H) 8.86 (d, J=1.25 Hz, 1 H) 10.08 (s, 1 H) | 264 | 0.79 | A |
| 48 | N-(1-(4-fluorophenyl)-1H-indol-5-yl)-acrylamide | 1H NMR (400 MHz, DMSO-d6) δ ppm 5.72 (dd, J=10.16, 2.13 Hz, 1 H) 6.25 (dd, J=17.07, 2.13 Hz, 1 H) 6.47 (dd, J=17.07, 10.16 Hz, 1 H) 6.67 (d, J=3.26 Hz, 1 H) 7.32 - 7.50 (m, 4 H) 7.57 - 7.67 (m, 3 H) 8.11 (d, J=1.76 Hz, 1 H) 10.06 (s, 1 H) | 281 | 1.22 | A |
| 49 | N-(1-(3,4-difluorophenyl)-1 H-indol-5-yl)-acrylamide | 1H NMR (400 MHz, DMSO-d6) δ ppm 5.73 (dd, J=10.04, 2.01 Hz, 1 H) 6.25 (dd, J=17.07, 2.01 Hz, 1 H) 6.46 (dd, J=17.07, 10.04 Hz, 1 H) 6.69 (d, J=3.26 Hz, 1 H) 7.40 (dd, J=8.91, 1.88 Hz, 1 H) 7.44 - 7.50 (m, 1 H) 7.54 (d, J=8.91 Hz, 1 H) 7.59 - 7.70 (m, 2 H) 7.71 - 7.81 (m, 1 H) 8.12 (d, J=1.88 Hz, 1 H) 10.08 (s, 1 H) | 299 | 1.26 | A |
| 50 | N-(1-(3-(methylsulfonyl) phenyl)-1H-indol-5-yl)-acrylamide | 1H NMR (400 MHz, DMSO-d6) δ ppm 3.34 (s, 3 H) 5.73 (dd, J=10.04, 2.01 Hz, 1 H) 6.26 (dd, J=16.94, 2.02 Hz, 1 H) 6.47 (dd, J=16.94, 10.04 Hz, 1 H) 6.76 (d, J=3.26 Hz, 1 H) 7.42 (dd, J=8.91, 1.88 Hz, 1 H) 7.59 (d, J=8.91 Hz, 1 H) 7.78 (d, J=3.26 Hz, 1 H) 7.82 - 7.88 (m, 1 H) 7.89 - 7.95 (m, 1 H) 7.97 - 8.03 (m, 1 H) 8.05 - 8.09 (m, 1 H) 8.15 (d, J=1.88 Hz, 1 H) 10.11 (s, 1 H) | 341 | 0.97 | A |
| 51 | N-(1-(4-cyanophenyl)-1 H-indol-5-yl)-acrylamide | 1H NMR (400 MHz, DMSO-d6) δ ppm 5.74 (dd, J=9.79, 2.26 Hz, 1 H) 6.26 (dd, J=16.81, 2.26 Hz, 1 H) 6.46 (dd, J=16.81, 9.79 Hz, 1 H) 6.77 (d, J=3.26 Hz, 1 H) 7.43 (dd, J=9.03, 2.01 Hz, 1 H) 7.66 (d, J=9.03 Hz, 1 H) 7.77 (d, J=3.26 Hz, 1 H) 7.83 (d, J=8.53 Hz, 2 H) 8.02 (d, J=8.53 Hz, 2 H) 8.13 (d, J=2.01 Hz, 1 H) 10.12 (s, 1 H) | 288 | 1.07 | A |

Compounds of formula (I) underwent biochemical studies in order to determine their capacity to inhibit YAP1/TAZ-TEAD or TEAD-dependent gene transcription.

### TEAD-luciferase reporter assay

Luciferase-based gene reporter assays were used, employing the TEAD responsive promoter element that are stably integrated to different human tumor cell lines in order to monitor YAP1-TEAD and TAZ-TEAD activity and to study YAP1/TAZ-TEAD activity modulation by small molecule compounds. Luciferase-based gene reporter assays employing the GAPDH promoter element and stably integrated to different human tumor cell lines are used as counter screen control cell lines.

Tumor cell lines with stable integration of the 8XGTIIC-Luciferase construct (Dupont et al., Nature 2011) or the GAPDH-Luciferase control plasmid, are seeded in culture medium in 96-well plates at a density of 8000 cells / well. Following an overnight incubation in a 37°C, 5% CO2 growth chamber, cells are treated with compounds described above at 10 doses ranging between 1 and 10000 nM for 48 hrs. After compound incubation, cells are lysed with Bright-Glo Luciferase Assay System (Promega E2620) and luciferase activity is measured using a luminescent plate reader.

The inhibitory activity of the compounds with respect to luciferase activity is given by the concentration which inhibits 50 % of activity of non-treated cells. IC50s are determined with a nonlinear regression model on XLfit software analysis (IDBS, UK).

The IC50 values for the compounds of the invention were generally less than 1 µM, more particularly between 1 and 550 nM and even more particularly between 1 and 100 nM, as indicated in the table below:

| example N° | TEAD IC50 (nM) | | example N° | TEAD IC50 (nM) | | example N° | TEAD IC50 (nM) |
|---|---|---|---|---|---|---|---|
| 1 | 24 | | 18 | 963 | | 35 | 2331 |
| 2 | 1087 | | 19 | 195 | | 36 | 4559 |
| 3 | 25 | | 20 | 7755 | | 37 | >10000 |
| 4 | 558 | | 21 | 35 | | 38 | 1500 |
| 5 | 61 | | 22 | 1404 | | 39 | 84 |
| 6 | 4044 | | 23 | 369 | | 40 | 10000 |
| 7 | 10000 | | 24 | 1000 | | 41 | 10000 |
| 8 | 296 | | 25 | 1000 | | 42 | 211 |
| 9 | 143 | | 26 | 357 | | 43 | 36 |
| 10 | 7224 | | 27 | 10000 | | 44 | 8 |
| 11 | 30 | | 28 | 10000 | | 45 | 106 |
| 12 | 241 | | 29 | 43 | | 46 | >10000 |
| 13 | 63 | | 30 | 10000 | | 47 | >10000 |
| 14 | 236 | | 31 | 20 | | 48 | >10000 |
| 15 | 496 | | 32 | 7842 | | 49 | >10000 |
| 16 | 31 | | 33 | 41 | | 50 | 4679 |
| 17 | 54 | | 34 | 10000 | | 51 | 313 |

It is therefore apparent that the compounds of formula (I) have an inhibitory activity of YAP1/TAZ-TEAD or TEAD-dependent gene transcription.

The compounds of formula (I) may thus be used as inhibitors of YAP1/TAZ-TEAD or TEAD-dependent gene transcription.

The compounds of formula (I) may thus be used as medicaments, especially medicaments which are inhibitors of YAP1/TAZ-TEAD or TEAD-dependent gene transcription.

Thus, according to another of its aspects, a subject of the invention is medicaments that comprise a compound of formula (I), or an addition salt thereof with a pharmaceutically acceptable acid.

These medicaments are employed therapeutically in the treatment of cancer, in particular in the treatment of breast, ovarian, uterine, prostate, lung, gastric, colorectal, bladder, pancreatic and liver cancers, sarcomas, esophageal, head and neck cancers, uveal melanoma, or glioma.

According to another of its aspects, the present invention relates to pharmaceutical compositions comprising as active principle, a compound of formula (I). These pharmaceutical compositions contain an effective dose of at least one compound of formula (I), or a pharmaceutically acceptable salt of the said compound.

These pharmaceutical compositions may also contain at least one pharmaceutically acceptable excipient.

The said excipients are chosen, according to the pharmaceutical form and the desired mode of administration, from the usual excipients known to those skilled in the art.

The compounds of formula (I) may be used in the treatment of pathologies involving YAP1/TAZ-TEAD or TEAD-dependent gene transcription inhibitors.

In particular, the compounds of formula (I) may be used as an anticancer agent, in particular for use in the treatment of breast, ovarian, uterine, prostate, lung, gastric, colorectal, bladder, pancreatic and liver cancers, sarcomas, esophageal, head and neck cancers, uveal melanoma, or glioma.

The compounds of formula (I) may also be used in the treatment of a patient who has exhibited resistance to prior anti-cancer therapy.

The present invention, according to another of its aspects, also provides a method of treating the pathologies indicated above.

Thus, described is also a method of treating cancer, in particular breast, ovarian, uterine, prostate, lung, gastric, colorectal, bladder, pancreatic and liver cancers, sarcomas, esophageal, head and neck cancers, uveal melanoma, or glioma, including administering to a subject in need thereof a therapeutically effective amount of at least one compound of formula (I) or a pharmaceutically acceptable salt thereof.

The compounds of formula (I) may also be used in a method if treating cancer in a patient who has exhibited resistance to prior anti-cancer therapy.

These compounds may be used in monotherapy or combination with radiotherapy or chemotherapy.

## Claims

1. Compound of the formula (I) 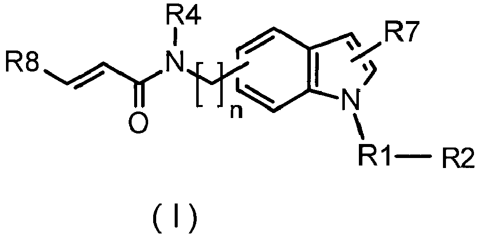 wherein
n is an integer chosen from 0 and 1
R1 is chosen from
- a single bond, and
- a (C1-C4) alkenyl group,
R2 is chosen from:
- a (C1-C4) alkyl group or substituted with one or more fluorine atoms,
- a (C1-C3) alkoxy group substituted with one or more fluorine atoms,
- a phenyl group unsubstituted or substituted with one or more R3 groups,
- a (C4-C8) cycloalkyl group unsubstituted or substituted with one or more R5 groups,
- a (C4-C8) heterocyclyl group unsubstituted or substituted with one or more R6 groups, and
- a NR9R10 group,
R3 is chosen from a
- a (C1-C4) alkyl group unsubstituted or substituted with one or more fluorine atoms,
- a cyclopropyl group,
- a halogen atom,
- a (C1-C3) alkoxy group unsubstituted or substituted with one or more fluorine atoms,
- a pentafluorosulfanyl group,
- a nitrile group,
- a (C1-C3) trialkylsilyl group,
- a (C1-C3) aklylsulfonyl group, and
- a phenyl group unsubstituted or substituted with a trifluoromethyl group,
R4 is chosen from a hydrogen atom and a (C1-C4) alkyl group,
R5 is chosen from a fluorine atom and a trifluoromethyl group,
R6 is chosen from
- a phenyl group unsubstituted or substituted with one or more fluorine atoms or one or more CF3 groups,
- a (C1-C4) alkyl group substituted with one or more fluorine atoms, and
- a fluorine atom,
R7 is chosen from
- a hydrogen atom,
- a nitrile group, and
- a (C1-C4) alkyl group,
R8 is chosen from a hydrogen atom and a (C1-C4) alkyl group unsubstituted or substituted with a di(C1-C4) alkylamino group,
R9 and R10 are identical or different and chosen from an (C1-C3) alkyl group unsubstituted or substituted with one or more fluorine atoms,
or a pharmaceutically acceptable salt thereof.

2. Compound according to claim 1, of the following formula 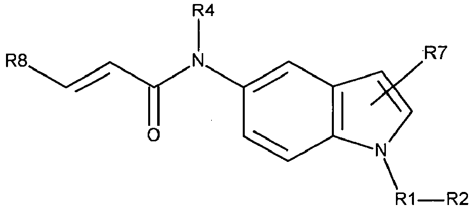 wherein R1, R2, R4, R7 and R8 are as defined in claim 1
or a pharmaceutically acceptable salt thereof.

3. Compound according to anyone of claim 1 or 2 wherein R4 is a hydrogen atom or a pharmaceutically acceptable salt thereof.

4. Compound according to anyone of claim 1 to 3 wherein R7 is a hydrogen atom or a pharmaceutically acceptable salt thereof.

5. Compound according to anyone of claim 1 to 4 wherein R8 is a hydrogen atom or a pharmaceutically acceptable salt thereof.

6. Compound according to anyone of claim 1 to 5 wherein
n is 0,
R1 is a single bond;
R2 is chosen from:
- a phenyl group unsubstituted or substituted with one or more R3 groups,
- a (C4-C8) cycloalkyl group unsubstituted or substituted with one or more R5 groups, and
- a (C4-C8) heterocyclyl group unsubstituted or substituted with one or more R6 groups,
or a pharmaceutically acceptable salt thereof.

7. Compound according to claim 6, wherein R2 is a phenyl group substituted with one or more R3 groups,
or a pharmaceutically acceptable salt thereof.

8. Compound according to anyone of claim 6 or 7, wherein R3 is a (C1-C4) alkyl group substituted with one or more fluorine atoms,
or a pharmaceutically acceptable salt thereof.

9. Compounds of formula (I) according to claim 1 which are selected from the following list:
N-[1-[[3-(trifluoromethyl)phenyl]methyl]indol-5-yl]prop-2-enamide
N-methyl-N-(1-(3-(trifluoromethyl)benzyl)-1H-indol-5-yl)acrylamide
N-(1-(5,5,5-trifluoropentyl)-1H-indol-5-yl)acrylamide
N-(1-(1-(3-(trifluoromethyl)phenyl)ethyl)-1H-indol-5-yl)acrylamide
N-(1-(3-(pentafluoro-lambda6-sulfanyl)benzyl)-1H-indol-5-yl)acrylamide
N-(1-(4-(pentafluoro-lambda6-sulfanyl)benzyl)-1H-indol-5-yl)acrylamide
N-(1-(4-(trifluoromethoxy)benzyl)-1H-indol-5-yl)acrylamide
N-(1-(3-(trifluoromethoxy)benzyl)-1H-indol-5-yl)acrylamide
N-(1-(3-iodobenzyl)-1H-indol-5-yl)acrylamide
N-(1-(2-fluoro-5-(trifluoromethyl)benzyl)-1H-indol-5-yl)acrylamide
N-(3-methyl-1-(3-(trifluoromethyl)benzyl)-1H-indol-5-yl)acrylamide
N-(1-(3-methoxybenzyl)-1H-indol-5-yl)acrylamide
N-(2-methyl-1-(3-(trifluoromethyl)benzyl)-1H-indol-5-yl)acrylamide
N-(3-cyano-1-(3-(trifluoromethyl)benzyl)-1H-indol-5-yl)acrylamide
N-(1-(3-cyclopropylbenzyl)-1H-indol-5-yl)acrylamide
N-(2,3-dimethyl-1-(3-(trifluoromethyl)benzyl)-1H-indol-5-yl)acrylamide
N-(1-(3-methylbenzyl)-1H-indol-5-yl)acrylamide
N-(1-Benzyl-1H-indol-5-yl)-acrylamide
N-(1-(3-(trifluoromethyl)benzyl)-1H-indol-4-yl)acrylamide
(E)-4-(dimethylamino)-N-(1-(3-(trifluoromethyl)benzyl)-1H-indol-4-yl)but-2-enamide
N-(1-(3-(trifluoromethyl)benzyl)-1H-indol-6-yl)acrylamide
N-(1-((4,4-difluorocyclohexyl)methyl)-1H-indol-5-yl)acrylamide
N-(1-((tetrahydro-2H-pyran-4-yl)methyl)-1H-indol-5-yl)acrylamide
N-(1-((4-(trifluoromethyl)cyclohexyl)methyl)-1H-indol-5-yl)acrylamide
N-(1-((1,1-difluorospiro[2.3]hexan-5-yl)methyl)-1H-indol-5-yl)acrylamide
N-(1-((3-fluorocyclopentyl)methyl)-1H-indol-5-yl)acrylamide
N-(1-(cyclohexylmethyl)-1H-indol-5-yl)acrylamide
N-(1-((3-(trifluoromethyl)cyclobutyl)methyl)-1H-indol-5-yl)acrylamide
N-(1-((3,3-difluorocyclopentyl)methyl)-1H-indol-5-yl)acrylamide
N-(1-([1,1'-biphenyl]-4-ylmethyl)-1 H-indol-5-yl)acrylamide
N-(1-((3'-(trifluoromethyl)-[1,1'-biphenyl]-4-yl)methyl)-1H-indol-5-yl)acrylamide
N-(1-((4'-(trifluoromethyl)-[1,1'-biphenyl]-4-yl)methyl)-1H-indol-5-yl)acrylamide
N-(1-((trans)-4-(trifluoromethyl)cyclohexyl)-1H-indol-5-yl)acrylamide
N-(1-((cis)-4-(trifluoromethyl)cyclohexyl)-1H-indol-5-yl)acrylamide
N-(1-((1-(4-(trifluoromethyl)phenyl)piperidin-4-yl)methyl)-1H-indol-5-yl)acrylamide
N-(1-(2-(methyl(2,2,2-trifluoroethyl)amino)ethyl)-1H-indol-5-yl)acrylamide
N-(1-(2-(2,2,2-trifluoroethoxy)ethyl)-1H-indol-5-yl)acrylamide
N-(1-(1-(2,2,2-trifluoroethyl)piperidin-4-yl)-1H-indol-5-yl)acrylamide
N-(1-(4,4-difluorocyclohexyl)-1H-indol-5-yl)acrylamide
N-(1-(1-(4-(trifluoromethyl)phenyl)piperidin-4-yl)-1 H-indol-5-yl)acrylamide
N-(1-(3-(3,3-difluoropyrrolidin-1-yl)propyl)-1H-indol-5-yl)acrylamide
N-(1-(4-(trimethylsilyl)benzyl)-1H-indol-5-yl)acrylamide
N-(1-(3-(trifluoromethyl)phenyl)-1H-indol-5-yl)acrylamide
N-(1-(4-(trifluoromethyl)phenyl)-1H-indol-5-yl)acrylamide
N-(1-(6-(trifluoromethyl)pyridin-3-yl)-1H-indol-5-yl)acrylamide
N-(1-(5-(trifluoromethyl)pyridin-2-yl)-1H-indol-5-yl)acrylamide
N-(1-(pyridin-3-yl)-1H-indol-5-yl)acrylamide
N-(1-(4-fluorophenyl)-1H-indol-5-yl)acrylamide
N-(1-(3,4-difluorophenyl)-1H-indol-5-yl)acrylamide
N-(1-(3-(methylsulfonyl)phenyl)-1H-indol-5-yl)acrylamide
N-(1-(4-cyanophenyl)-1H-indol-5-yl)acrylamide
or a pharmaceutically acceptable salt thereof.

10. Medicament, **characterized in that** it comprises a compound of formula (I) according to any one of claim 1 to 9, or a pharmaceutically acceptable salt thereof.

11. Pharmaceutical composition, **characterized in that** it comprises a compound of formula (I) according to any one of claim 1 to 9, or a pharmaceutically acceptable salt thereof.

12. Compound of formula (I) according to any one of claim 1 to 9, for use as an anticancer agent.

13. Compound of formula (I) according to any one of claim 1 to 9, for use in the treatment of breast, ovarian, uterine, prostate, lung, gastric, colorectal, bladder, pancreatic and liver cancers, sarcomas, esophageal, head and neck cancers, uveal melanoma, or glioma.

14. Compound of formula (I) according to any one of claim 1 to 9, for use in the treatment of a patient who has exhibited resistance to prior anti-cancer therapy.
